(19) Europäisches Patentamt
European Patent Office
Office européen des brevets

(11) Numéro de publication: **0 414 606 B1**

# (12) FASCICULE DE BREVET EUROPEEN

(45) Date de publication de fascicule du brevet: **02.11.94**

(51) Int. Cl.$^5$: **C07J 41/00**, C07J 63/00, A61K 31/56

(21) Numéro de dépôt: **90402328.0**

(22) Date de dépôt: **22.08.90**

(54) **Nouveaux acides oméga-phénylamino alcanoiques substitués sur le noyau aromatique par un radical dérivé de 19-nor stéroides, leurs sels, leur procédé de préparation et les intermédiaires de ce procédé, leur application à titre de médicaments et les compositions les renfermant.**

(30) Priorité: **23.08.89 FR 8911173**

(43) Date de publication de la demande:
**27.02.91 Bulletin 91/09**

(45) Mention de la délivrance du brevet:
**02.11.94 Bulletin 94/44**

(84) Etats contractants désignés:
**AT BE CH DE DK ES FR GB GR IT LI LU NL SE**

(56) Documents cités:
**EP-A- 0 308 345**
**WO-A-87/05908**
**DE-A- 3 619 413**
**GB-A- 2 160 873**

(73) Titulaire: **ROUSSEL-UCLAF**
**35, Boulevard des Invalides**
**F-75007 Paris (FR)**

(72) Inventeur: **Moguilewsky, Martine**
**54, rue Notre Dame de Lorette**
**F-75009 Paris (FR)**
Inventeur: **Nedelec, Lucien**
**45, Boulevard de l'Ouest**
**F-93340 Le Raincy (FR)**
Inventeur: **Nique, François**
**7, Allée Pierre Brossolette**
**F-93320 Pavillons Sous Bois (FR)**
Inventeur: **Philibert, Daniel**
**16, rue Chevalier**
**F-94210 La Varenne Saint Hilaire (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 414 606 B1

Rank Xerox (UK) Business Services
(3.10/3.09/3.3.3)

## Description

La présente invention concerne des nouveaux acides oméga-phénylamino alcanoïques substitués sur le noyau aromatique par un radical dérivé de 19-nor stéroïdes, leurs sels, leur procédé de préparation et les intermédiaires de ce procédé, leur application à titre de médicaments et les compositions les renfermant.

L'invention a pour objet les produits de formule générale (I) :

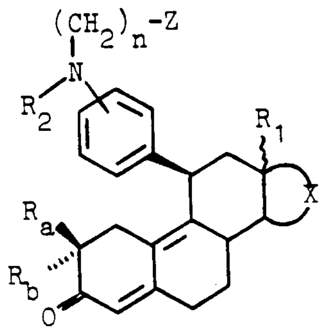

(I)

dans laquelle $R_1$ représente un radical hydrocarboné aliphatique renfermant de 1 à 8 atomes de carbone, $R_a$ et $R_b$ identiques ou différents représentent un atome d'hydrogène, un radical alkyle renfermant de 1 à 4 atomes de carbone, $R_2$ représente un atome d'hydrogène ou un radical alkyle renfermant de 1 à 12 atomes de carbone et éventuellement substitué, n représente une des valeurs de 1 à 6, Z représente un groupe carboxy libre ou salifié sous forme d'un sel de métal alcalin ou alcalino-terreux, d'un sel d'ammonium ou d'un sel d'amine, X représente le reste d'un cycle pentagonal ou hexagonal éventuellement substitué et éventuellement porteur d'une insaturation, et le trait ondulé en position 13 signifie que $R_1$ peut se trouver en position alpha ou béta.

$R_1$ représente de préférence un radical alkyle, linéaire ou ramifié, renfermant de 1 à 4 atomes de carbone comme le radical méthyle, éthyle, propyle, isopropyle ou butyle.

On préfère les produits dans lesquels $R_1$ se trouve en position béta.

Quand $R_a$ ou $R_b$ représente un radical alkyle, il s'agit de préférence du radical méthyle mais $R_a$ ou $R_b$ peut également représenter un radical éthyle, propyle, isopropyle ou butyle.

Quand $R_2$ représente un radical alkyle éventuellement substitué, il s'agit de préférence du radical méthyle, éthyle ou propyle éventuellement substitué par un groupe diméthylamino ou diéthylamino ou encore par un radical pyrrolidinyle, pipéridinyl, pipérazinyle ou morpholinyle.

Lorsque Z représente un groupe carboxy salifié, il s'agit de préférence du sel de sodium, de potassium, de calcium, de magnésium, d'ammonium ou d'un sel d'amine utilisée dans la fabrication des médicaments, comme les sels de lysine, d'arginine, de cystéïne, de bétaïne, de carnitine, de méglumine, de quinine, de sarcosine, de procaïne, d'histidine ou de N-méthyl glucamine.

L'invention a notamment pour objet les produits de formule générale (I) telle que définie précédemment et répondant à la formule (I') :

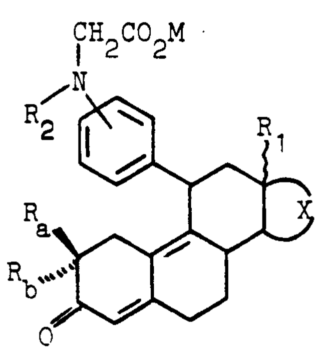

(I')

dans laquelle M représente un atome d'hydrogène ou un atome de sodium et principalement les produits de formule générale (I) dans laquelle X représente un reste de formule

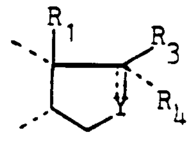

dans lequel $R_1$ conserve la même signification que précédemment, le trait pointillé en 16-17 symbolise la présence éventuelle d'une double liaison, Y représente un radical

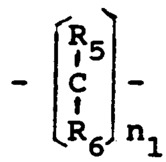

dans lequel $n_1$ représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$ identique ou différent de $R_5$ peut prendre l'une des valeurs indiquées pour $R_5$ et peut également représenter un radical hydroxyle, $R_3$ et $R_4$, identiques ou différents, représentent

soit un atome d'hydrogène,

soit un radical choisi dans le groupe formé par les radicaux OH, $Oalc_1$, O-CO-$alc_2$ dans lesquels $alc_1$ et $alc_2$ représentent un radical alkyle renfermant de 1 à 8 atomes de carbone ou aralkyle renfermant de 7 à 15 atomes de carbone,

soit un radical alkyle, alkényle ou alkynyle renfermant de 2 à 8 atomes de carbone et éventuellement substitué,

soit un radical

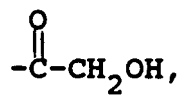

soit un radical $-COCH_2OCOalc_3$ dans lequel $alc_3$ représente un radical alkyle renfermant de 1 à 8 atomes de carbone éventuellement substitué ou un radical aralkyle renfermant de 7 à 15 atomes de carbone,

soit un radical CO-$CO_2$H,

soit un radical CO-CO-$alc_4$ dans lequel $alc_4$ représente un radical alkyle renfermant de 1 à 8 atomes de carbone,

soit un radical

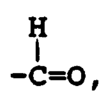

soit un radical

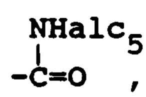

dans lequel $alc_5$ représente un radical alkyle renfermant de 1 à 8 atomes de carbone ou un radical aralkyle renfermant de 7 à 15 atomes de carbone,

soit un radical -C≡N,

soit $R_3$ et $R_4$ forment ensemble avec l'atome de carbone auquel ils sont liés un radical

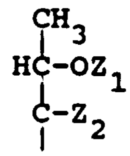

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone,
soit $R_3$ et $R_4$ forment ensemble avec l'atome auquel ils sont liés,
ou bien un radical

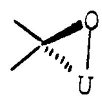

dans lequel U représente un radical bivalent $-(CH_2)n_2$ dans lequel $n_2$ représente un entier égal à 1, 2, 3 ou 4 ou un radical bivalent $-CH=CH-(CH_2)n_3-$ dans lequel $n_3$ représente un entier égal à 1 ou 2,
ou bien un radical

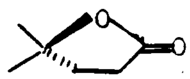

Lorsque $R_5$ ou $R_6$ représente un radical alkyle, il s'agit de préférence du radical méthyle ou éthyle.

Lorsque $R_5$ ou $R_6$ représente un radical alkényle, il s'agit de préférence du radical vinyle, isopropényle ou allyle.

Lorsque $R_5$ ou $R_6$ représente un radical alkynyle, il s'agit de préférence du radical éthynyle ou propynyle.

Lorsque $R_5$ ou $R_6$ représente un radical aryle ou aralkyle, il s'agit de préférence du radical phényle ou benzyle.

Lorsque $R_3$ ou $R_4$ représente un radical $Oalc_1$ ou $OCOalc_2$, $alc_1$ et $alc_2$ représentent de préférence un radical méthyle, éthyle, n-propyle, butyle, pentyle, hexyle ou benzyle. Lorsque $R_3$ ou $R_4$ représente un radical alkyle, alkényle ou alkynyle éventuellement substitué, il s'agit de préférence d'un groupe $-C\equiv C-W$, $-CH=CH-W$ ou $-CH_2-CH_2-W$ dans lesquels W représente un atome d'hydrogène, un atome d'halogène, un radical trialkylsilyle renfermant de 3 à 12 atomes de carbone, un radical alkyle renfermant de 1 à 6 atomes de carbone, linéaire ou ramifié ou un radical phényle, lesdits radicaux alkyle et phényle étant éventuellement substitués.

$alc_3$, $alc_4$ et $alc_5$ représentent de préférence une des valeurs préférentielles de $alc_1$ et $alc_2$.

Très préférentiellement, Y représente un radical méthylène.

L'invention a particulièrement pour objet les produits de formule générale (I) telle que définie précédemment dans laquelle le cycle D du noyau stéroïde ne porte pas d'insaturation $R_5$ et $R_6$ représentent un atome d'hydrogène et $n_1$ est égal à 1 et tout particulièrement ceux répondant à la formule (I"):

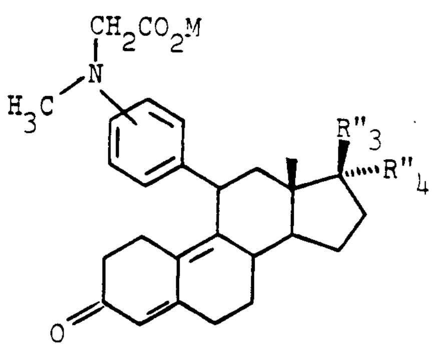

(I")

dans laquelle M est tel que défini précédemment et soit $R''_3$ représente un radical hydroxy et dans ce cas $R''_4$ représente ou bien un groupe

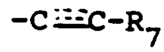

dans lequel les traits pointillés indiquent la présence éventuelle d'une deuxième ou d'une troisième liaison et $R_7$ représente un atome d'hydrogène, un atome d'halogène, un radical triméthylsilyle ou un radical méthyle éventuellement substitué par un ou plusieurs atomes d'halogène, un radical hydroxy ou par un groupe alkyloxy, alkylthio ou alkylamino renfermant de 1 à 4 atomes de carbone, dialkylamino renfermant de 2 à 8 atomes de carbone ou trialkylsilyle renfermant de 3 à 12 atomes de carbone ou bien un radical 2-propynyle ou 2-propényle, soit $R''_3$ et $R''_4$ représentent ensemble avec l'atome auquel ils sont liés un radical

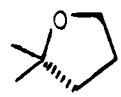

ou

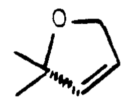

Parmi les composés préférés de l'invention, on peut donc naturellement citer les produits décrits ci-après dans les exemples et tout spécialement le sel de sodium de l'acide [[4-[17béta hydroxy 3-oxo 17alpha (1-propynyl) estra 4,9-dièn 11-béta-yl] phényl] méthylamino] acétique.

L'invention a également pour objet un procédé de préparation des produits de formule (I) telle que définie précédemment, caractérisé en ce que les produits de formule ($II_a$) et ($II_b$) :

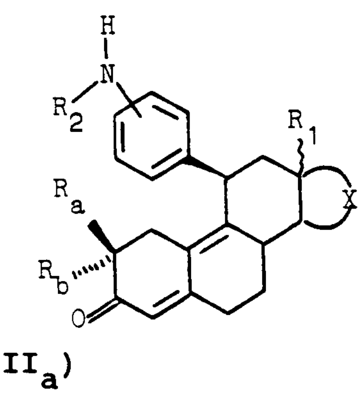

($II_a$)

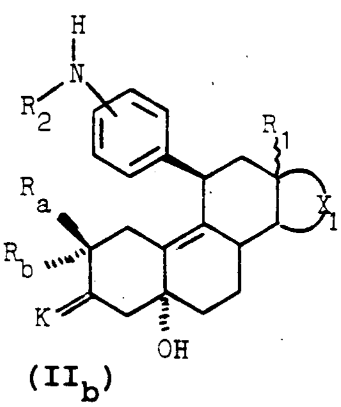

($II_b$)

dans lesquels $R_1$, $R_2$, $R_a$, $R_b$ et X sont tels que définis précédemment, $X_1$ a la signification indiquée précédemment pour X ainsi que ces valeurs dans lesquelles les fonctions réactives sont protégées et K représente une fonction oxo protégée, sont soumis en présence d'une base à l'action d'un halogéno-ester de formule (III) :

$Hal-(CH_2)n-COOR_7$ (III)

dans laquelle Hal représente un atome d'halogène, n a la signification indiquée précédemment et $R_7$ représente un radical alkyle renfermant de 1 à 4 atomes de carbone et éventuellement substitué par un ou plusieurs radicaux phényle, pour obtenir respectivement les produits de fomules ($IV_a$) et ($IV_b$) :

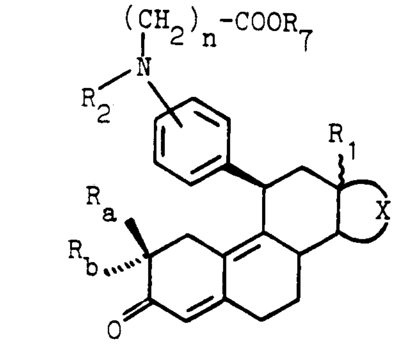

(IV$_a$)

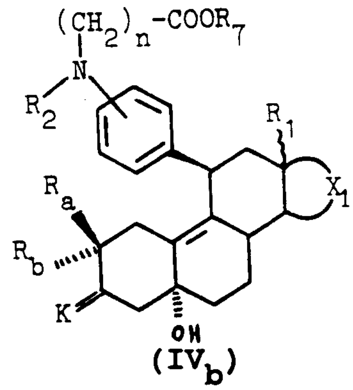

(IV$_b$)

produits de formule (IV$_b$) que l'on soumet à une réaction de déshydratation et de déprotection éventuelle des fonctions réactives protégées pour obtenir les produits de formule (IV$_a$) et produits de formule (IV$_a$) que l'on soumet à un traitement basique puis, si désiré, acide pour obtenir les produits de formule (I).

Dans un mode préférentiel d'exécution du procédé, les produits de formules (II"$_a$) et (II"$_b$) :

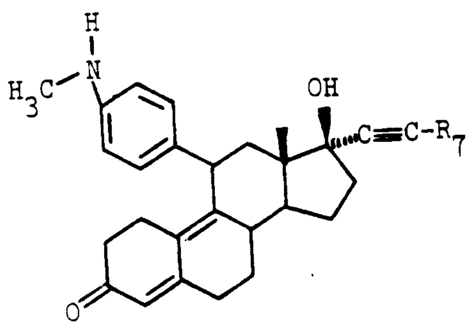

(II"$_a$)

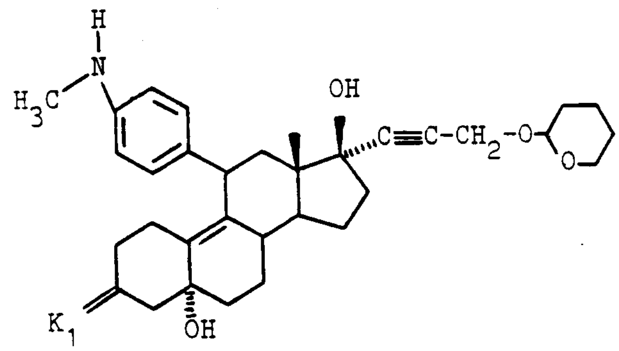

(II"$_b$)

dans laquelle $K_1$ représente un groupe oxo protégé sous forme d'un radical éthylènedioxy ou d'un acétal diméthylique sont soumis à l'action du bromoacétate d'éthyle en présence d'une base azotée pour obtenir respectivement les produits de formules (IV"$_a$) et (IV"$_b$) :

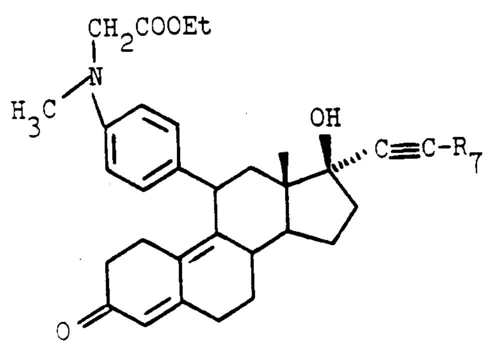

(IV"$_a$)

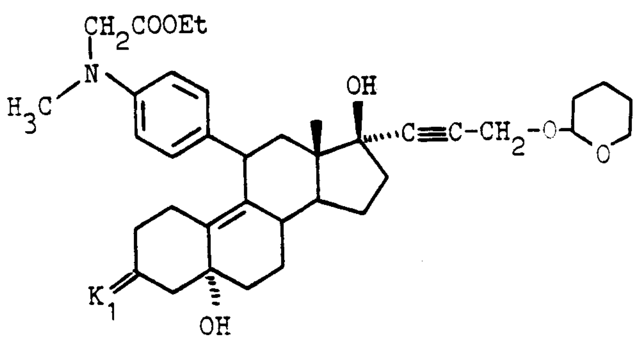

(IV"$_b$)

produits de formules (IV"$_a$) et (IV"$_B$) qui si désiré sont hydrogénés pour obtenir respectivement suivant le catalyseur utilisé soit les produits de formules (V"$_a$) et (V"$_b$) :

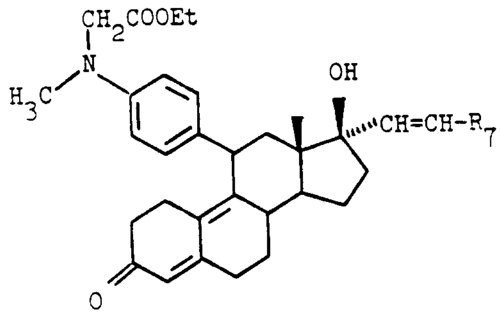

(V"$_{a}$)

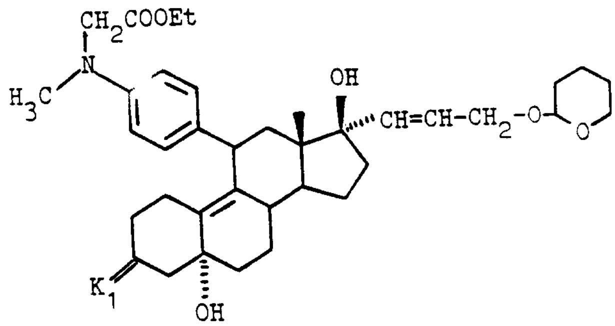

(V"$_{b}$

soit les produits de formule (VI"$_{a}$) et (VI"$_{b}$) :

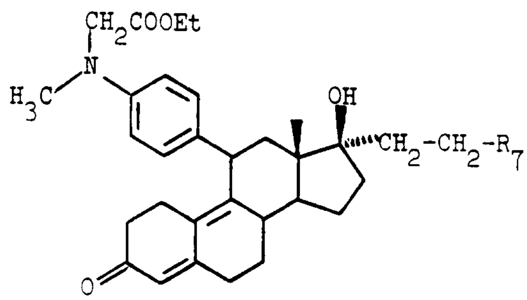

(VI"$_{a}$)

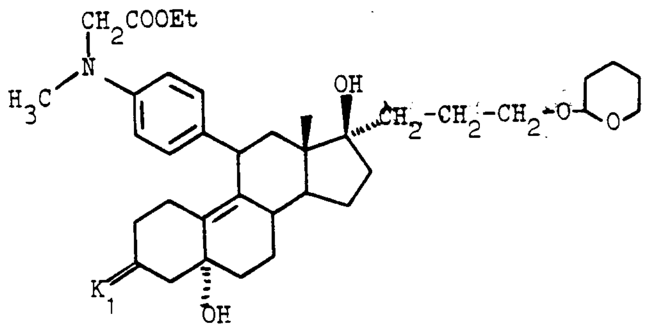

(VI"$_{b}$)

produits de formule (V"$_{b}$) et (VI"$_{b}$) qui par traitement acide conduisent aux produits de formules (V"$_{c}$) et (VI"$_{c}$) :

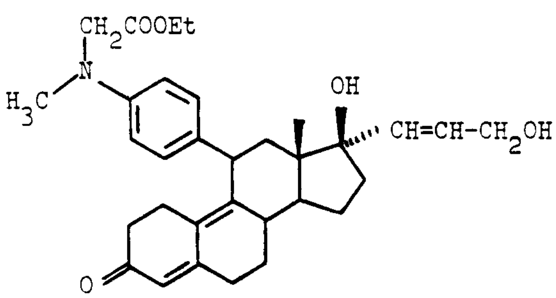

(V"$_{c}$)

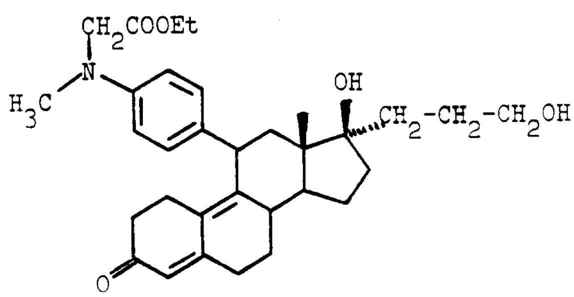

(VI"$_{c}$)

produits de formules (V"$_{c}$) et (VI"$_{c}$) que si désiré l'on fait réagir avec du chlorure de tosyle dans la pyridine pour obtenir respectivement les produits de formules (V"$_{d}$) et (VI"$_{d}$) :

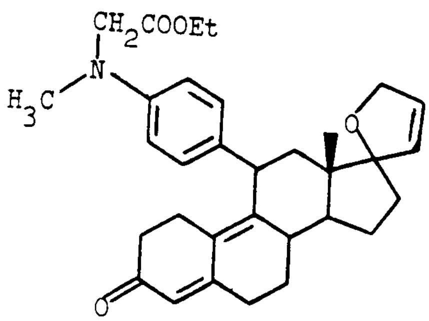

(V"$_d$)

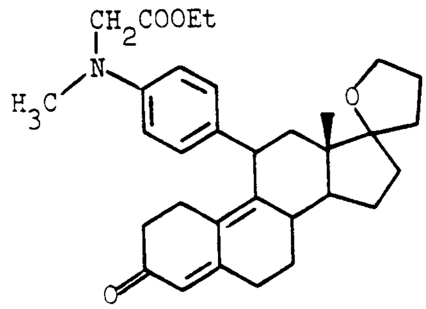

(VI"$_d$)

produits de formules (IV"$_a$), (V"$_a$), (V"$_c$), VI"$_a$), (VI"$_c$, (V"$_d$) et (VI"$_d$) que l'on traite avec une solution hydroalcoolique de soude puis si désiré par l'acide chlorhydrique pour obtenir les produits de formule (I").

La base azotée utilisée avec le bromoacétate d'éthyle est de préférence la triéthylamine.

L'hydrogénation des liaisons triples en liaisons doubles se fait en présence d'un catalyseur tel que l'hydroxyde de palladium sur sulfate de baryum ; l'hydrogénation complète en liaison simple se fait de préférence en présence de [chloro-tris (triphénylphosphine) rhodium].

Les produits de formule (III) utilisés pour la mise en oeuvre du procédé de préparation des produits de formule (I) sont pour certains des produits commercialisés ; les autres peuvent être préparés par des méthodes connues de l'homme de l'art, comme par exemple celle décrite dans MOUREU, MURAT et TAMPIER, comptes rendus de l'Académie des Sciences, 172, p. 1268 ; Annales de Chimie [9] 15, p. 233.

Les produits de formules (II$_a$) et (II$_b$) sont connus de manière générale ; leur préparation est décrite dans les brevets européens 0 262 188, 0 097 572, 0 057 115 et les brevets français 2 566 779 et 2 620 707.

Les produits de formule générale (I) possèdent d'intéressantes propriétés pharmacologiques.

L'étude des produits sur les récepteurs hormonaux a permis de mettre en évidence des activités progestomimétiques ou antiprogestomimétiques, androgènes ou anti-androgènes.

Les produits de la présente demande de brevet peuvent également présenter des propriétés antiprolifératives, anti-glucocorticoïdes,anti-estrogènes et/ou estrogènes.

Ces produits peuvent donc être utilisés comme médicaments pour lutter principalement contre les effets secondaires des glucocorticoïdes ; ils permettent de lutter également contre les troubles dus à une hypersécrétion de glucocorticoïdes et notamment contre le vieillissement en général et plus particulièrement contre l'hypertension, le glaucome, l'athérosclérose, l'ostéoporose, le diabète, l'obésité ainsi que la dépression de l'immunité et l'insomnie.

Les produits qui possèdent des propriétés antiprogestomimétiques peuvent être utilisés pour préparer des contraceptifs originaux ou comme agents d'interruption de grossesse.

Les produits objets de la présente demande de brevet peuvent aussi être utilisés comme inducteurs de règles chez la femme et plus généralement chez les animaux femelles à sang chaud.

Ces produits sont alors administrés pendant les périodes où la progestérone joue un rôle physiologique essentiel, c'est-à-dire notamment pendant la phase lutéale du cycle, au moment de la nidation (ou implantation de l'embryon) et pendant la grossesse. Une méthode de contraception selon l'invention consiste à administrer à la femme un au moins des produits de formule (I) pendant 1 à 5 jours se situant préférentiellement à la fin du cycle. Ces produits sont administrés alors préférentiellement par la voie orale ou in vagino mais ils peuvent également être utilisés par la voie parentérale.

Les produits objets de la présente demande de brevet peuvent également être utilisés par la voie endonasale.

Ces produits qui possèdent des propriétés antiprogestomimétiques peuvent également être utilisés contre les dérèglements hormonaux et, par ailleurs, ils peuvent présenter un intérêt dans le traitement des tumeurs hormono-dépendantes.

Leurs actions sur les sécrétions hypophysaires rendent les produits utilisables dans la ménopause.

Ces produits peuvent également être utilisés dans la synchronisation de l'estrus chez les animaux d'élevage, en particulier les bovins et les ovins.

Ces produits peuvent également être utilisés pour contrôler la fertilité des animaux tels que chiens ou chats.

Les produits objets de la présente demande de brevet peuvent également présenter des propriétés progestomimétiques et peuvent ainsi être utilisés dans le traitement des aménorrhées, des dysménorrhées et des insuffisances lutéales.

Ces produits qui présentent des propriétés antiandrogènes peuvent être utilisés dans le traitement des hypertrophies et du cancer de la prostate, de l'hyperandrogénie, de l'anémie, de l'hirsutisme et de l'acné. Ils peuvent également être utilisés pour la contraception chez l'homme.

Enfin les produits objets de la présente demande de brevet qui présentent des propriétés antiprolifératives, anti-estrogènes et/ou estrogènes peuvent être utilisables dans le traitement des carcinomes hormonodépendants, comme par exemple les carcinomes mammaires et leurs métastases. Ces propriétés rendent les produits également utilisables dans le traitement des tumeurs bénignes du sein.

Les propriétés estrogènes que peuvent également présenter lesdits produits les rendent utilisables également dans le traitement des troubles liés à une hypofolliculinie, par exemple les aménorrhées, les dysménorrhées, les avortements répétés, les troubles prémenstruels ainsi que le traitement de la ménopause.

L'invention a donc pour objet, à titre de médicaments, les produits de formule (I) ou leurs sels pharmaceutiquement acceptables objets de la présente demande de brevet et plus particulièrement ceux de formules (I′) et (I″) ou leurs sels pharmaceutiquement acceptables.

L'invention a tout particulièrement pour objet, à titre de médicaments, le sel de sodium de l'acide [[4-[17béta hydroxy 3-oxo 17alpha (1-propynyl) estra 4,9-dièn 11-béta-yl] phényl] méthylamino] acétique.

La posologie utile varie en fonction de l'affection à traiter et de la voie d'administration ; elle peut varier par exemple de 10 mg à 1 g par jour chez l'adulte par voie orale.

Les nouveaux produits objets de la présente demande de brevet, tels que définis précédemment peuvent être employés pour préparer des compositions pharmaceutiques renfermant, à titre de principe actif, l'un au moins desdits produits pharmaceutiquement acceptables .

Ces produits sont utilises par voie digestive, parentérale ou locale. Ils peuvent être prescrits sous forme de comprimés simples ou dragéifiés, de gélules, de granulés, de suppositoires, d'ovules, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, les dispersants ou émulsifiants, les conservateurs.

De plus, les produits de formule générale (I) dans laquelle Z représente un groupe carboxy salifié et tout particulièrement les produits de formule (I′) et (I″) dans lesquelles M représente un atome de sodium sont très solubles dans l'eau, ce qui permet leur utilisation sous forme de solutés buvables, nasaux ou auriculaires, de collyres, d'aérosols, de solutions injectables IM ou IV ou de capsules ; la solubilité du produit de l'exemple 7 est ainsi supérieure à 59 g pour 100 ml d'eau.

L'invention a donc pour objet les compositions pharmaceutiques renfermant comme principe actif au moins un produit objet de la présente demande de brevet.

L'invention a également pour objet, à titre de produits industriels nouveaux, les produits de formules ($IV_a$) et ($IV_b$) telles que définies précédemment.

Les exemples et l'étude biologique suivants illustrent l'invention sans toutefois la limiter.

**Exemple 1 : Sel de sodium de l'acide [[4-[17béta hydroxy 3-oxo 17alpha (1- propynyl) estra 4,9-dièn 11béta-yl] phényl] méthylamino] acétique.**

**Stade A :** [[4-[17béta hydroxy 3-oxo 17alpha (1-propynyl) estra 4,9-dièn 11béta-yl] phényl] méthylamino] acétate d'éthyle.

1,66 g de 17béta hydroxy 11-[4-(méthylamino) phényl] 17alpha (1-propynyl) estra 4,9-dièn-3-one sont dissous avec 3,2 cm3 de triéthylamine dans 60 cm3 de benzène ; à la solution chauffée au reflux sous azote, sont ajoutés 3,2 cm3 de bromoacétate d'éthyle. Après refroidissement, le milieu réactionnel est dilué avec une solution aqueuse de bicarbonate de sodium, extrait la phase organique lavée à l'eau, séchée puis distillée. On chromatographie sur silice avec un mélange cyclohexane-acétate d'éthyle (1-1) et recueille 2 g de produit que l'on recristallise dans un mélange chlorure de méthylène-éther isopropylique pour obtenir 1,7 g du produit recherché. F = 110 °C environ.

$[alpha]_D$ = +120° (c = 1% dans $CHCl_3$).

Rf = 0,36 [chromatographie sur couche mince ; support $SiO_2$ ; éluant : mélange cyclohexane-acétate

d'éthyle (1-1)].

**Stade B :** Sel de sodium de l'acide [[4-[17béta hydroxy 3-oxo 17alpha (1-propynyl) estra 4,9-dièn 11béta-yl] phényl] méthylamino] acetique.

0,5 g de l'ester préparé au stade précédent est traité par 1 cm3 de soude normale dans 10 cm3 d'éthanol absolu à température ambiante. Après 15 heures de repos à cette température, la solution est évaporée sous pression réduite, le résidu obtenu est dissous dans le minimum de méthanol à 50% dans l'eau et chromatographié Lichrosorb KC 18 [R]. On élue d'abord avec du méthanol à 70% puis avec du méthanol à 50% d'eau. 0,35 g du produit recherché est ainsi séparé.
$[alpha]_D$ = +137° (c = 1M dans EtOH).
Rf = 0,6 [chromatographie sur couche mince ; support Silice KC 18 Whatman[R]; éluant : solution aqueuse de méthanol à 70%).

**Exemple 2 : Sel de sodium de l'acide [[4-[17béta hydroxy 17alpha (3-hydroxy 1-propynyl) 3-oxo estra 4,9-dièn 11béta-yl] phényl] méthylamino] acétique.**

**Stade A :** [[4-[3,3-[1,2-éthanediylbis (oxy)] 5alpha, 17béta dihydroxy 17alpha [3-(tétrahydro 2-pyrranyloxy) 1-propynyl) estr 9-èn 11béta-yl] phényl] méthylamino] acétate d'éthyle.

7,1 g du (éthanediyl) acétal cyclique de 5alpha, 17béta dihydroxy 11béta [4-(méthylamino) phényl] 17alpha [3-(tétrahydro 2-pyrranyloxy) 1-propynyl] estr 9-èn-3-one sont dissous avec 8 cm3 de triéthylamine dans 140 cm3 de tétrahydrofuranne ; à la solution au reflux on ajoute en 50 minutes une solution de 5 cm3 de bromoacétate d'éthyle dans 25 cm3 de tétrahydrofuranne ; après 3 heures d'agitation au reflux, sont ajoutés de nouveau 1 cm3 de triéthylamine puis 0,5 cm3 de bromoacétate d'éthyle et le milieu réactionnel est laissé 1 heure au reflux puis, après refroidissement, versé dans une solution aqueuse de bicarbonate de sodium et extrait à l'acétate d'éthyle. La phase organique est lavée à l'eau, séchée et évaporée sous pression réduite ; le résidu obtenu est chromatographié sur silice en éluant par le mélange cyclohexane-acétate d'éthyle (1-1). 5,6 g du produit recherché sont ainsi recueillis.
Rf = 0,32 [chromatographie sur couche mince ; support Silice ; éluant : mélange cyclohexane-acétate d'éthyle (1-1)].

**Stade B :** [[4-[17béta hydroxy 17alpha (3-hydroxy 1-propynyl) 3-oxo estra 4,9-dièn 11béta-yl] phényl] méthylamino] acétate d'éthyle.

0,5 g du produit obtenu au stade précédent sont dissous dans 5 cm3 de méthanol avec 5 cm3 d'acide chlorhydrique 2N et la solution ainsi obtenue est laissée 2 heures à température ambiante puis neutralisée avec une solution aqueuse de bicarbonate de sodium et extrait au chlorure de méthylène ; la phase organique est lavée à l'eau, séchée et évaporée. On recueille un produit brut qui est recristallisé dans l'éther sulfurique pour donner 0,32 g du produit recherché
F ≃ 110°-140°C.
$[alpha]_D$ = +109° (c = 1% dans $CHCl_3$).
Rf = 0,53 [chromatographie sur couche mince ; support : silice ; éluant : acétate d'éthyle.

**Stade C :** Sel de sodium de l'acide [[4-[17béta hydroxy 17alpha (3-hydroxy 1-propynyl) 3-oxo estra 4,9-dièn 11béta-yl] phényl] méthylamino] acétique.

0,32 g du produit préparé au stade précédent sont dissous avec 1,5 cm3 de méthanol et 0,55 cm3 de soude normale à 60°C sous azote. Après 80 minutes à cette température, le mélange est refroidi puis chromatographié sur Bondapack C 18 [R] en éluant par les mélanges méthanol-eau (25-75) puis (30-70). 280 mg de produit recherché sont ainsi obtenus.
$[alpha]_D$ = +133° (c = 1% dans EtOH).

**Exemple 3 : Sel de sodium de l'acide (Z) [[4-[17béta hydroxy 17alpha (3-hydroxy 1-propényl) 3-oxo estra 4,9-dièn 11béta-yl] phényl] méthalamino] acétique.**

**Stade A :** (Z) [[4-[3,3-[1,2-éthanediylbis (oxy)] 5alpha, 17béta dihydroxy 17alpha (3-(tétrahydro 2-pyrranyloxy) 1-propényl] estr 9-èn 11béta-yl] phényl] methylamino] acétate d'éthyle et son isomère (E).

0,8 g du produit préparé au stade A de l'exemple 2 sont dissous dans 16 cm3 d'acétate d'éthyle avec 0,3 ml de pyridine ; 12 cm3 d'hydroxyde de palladium sur sulfate de baryum à 10% sont ajoutés à la solution et le tout est soumis à une hydrogénation pendant 40 minutes (absorption de 27,1 cm3 d'hydrogène). On purge à l'azote, essore le catalyseur et évapore le filtrat. On obtient 0,8 g d'un mélange d'isomères (E) et (Z) que l'on sépare par chromatographie sur silice en éluant avec un mélange cyclohexaneacétate d'éthyle (1-1). 0,655 g de l'isomère (Z) et 0,065 g d'isomère (E) sont ainsi recueillis.

**Stade B :** (Z) [[4-[17béta hydroxy 17alpha (3-hydroxy 1-propényl) 3-oxo estra 4,9-dièn 11béta-yl] phényl] méthylamino] acétate d'éthyle.

6,3 g du produit (isomère (Z)) préparé au stade précédent sont dissous dans 60 cm3 d'éthanol ; 60 cm3 d'acide chlorhydrique 2N sont ensuite ajoutés, la solution est laissée pendant 5 heures à température ambiante puis versée prudemment dans une solution aqueuse de bicarbonate de sodium et extraite au chlorure de méthylène. La phase organique est lavée à l'eau, séchée puis évaporée sous pression réduite. On obtient 5,27 g d'un mélange que l'on chromatographie sur silice en éluant avec un mélange cyclohexane-acétate d'éthyle (2-8). On obtient 0,486 g de produit attendu sont ainsi recueillis. F = 163 °C.
$[alpha]_D$ = +20° (c = 1% dans le chloroforme).
Rf = 0,29 [chromatographie sur couche mince ; support : silice ; éluant : mélange cyclohexane-acétate d'éthyle (2-8)].

**Stade C :** Sel de sodium de l'acide (Z) [[4-[17béta hydroxy 17alpha (3- hydroxy 1-propényl) 3-oxo estra 4,9-dièn 11bétayl] phényl] methylamino] acétique.

0,78 g du produit préparé au stade précédent sont dissous dans 3,5 cm3 de méthanol et 1,5 cm3 de soude normale à 60 °C sous pression réduite. Le chauffage est maintenu pendant 1 heure puis la solution est refroidie et chromatographiée sur Bondapack $^R$ KC 18 en éluant avec le mélange méthanol-eau (4-6). Les éluats sont lyophilisés. On obtient 0,712 g du produit recherché.
$[alpha]_D$ = +202° (c = 1% dans EtOH).
Rf = 0,60 [chromatographie sur couche mince ; support : silice KC 18 Whatman $^R$ ; éluant : méthanol-solution aqueuse d'acétate d'éthyle 0,05M (65-35)].

**Exemple 4 : Sel de sodium de l'acide [[4-[17béta hydroxy 17alpha (3-hydroxy propyl) 3-oxo estra 4,9-dièn 11béta-yl] phényl] méthylamino] acétique.**

**Stade A :** [[4-[3,3-[éthanediylbis (oxy)] 5alpha, 17béta dihydroxy 17alpha [3-(tétrahydro 2-pyrranyloxy) propyl) estr 9-èn 11béta-yl] phényl] méthylamino] acétate d'éthyle.

5,6 g du produit préparé au stade A de l'exemple 2 sont dissous dans 70 cm3 de toluène ; 1,38 g du réactif de Wilkinson [chlorotris (triphénylphosphine) rhodium] sont ajoutés et on hydrogène jusqu'à saturation ; après évaporisation sous pression réduite 7,2 g d'une résine rouge sont obtenues qui, après chromatographie sur silice en éluant avec le mélange cyclohexane-acétate d'éthyle (1-1), conduisent à 5,6 g de produit recherché.

**Stade B :** [[4-[17béta hydroxy 17alpha (3-hydroxy propyl) 3-oxo estra 4,9- dièn 11béta-yl] phényl] méthylamino] acétate d'éthyle.

Les 5,6 g du produit obtenu au stade précédent sont dissous dans 50 cm3 de méthanol avec 50 cm3 d'acide chlorhydrique 2N. La solution est laissée 2 heures à température ambiante puis versée dans une solution aqueuse de bicarbonate de sodium et extraite au chlorure de méthylène. La phase organique est lavée à l'eau, séchée et évaporée. Le résidu obtenu est chromatographié sur silice en éluant avec le mélange cyclohexane-acétate d'éthyle (2-8). Après recristallisation dans l'éther sulfurique, on obtient 2,286 g du produit recherché. F = 100 °C.

$[alpha]_D$ = +175° (c = 1% dans $CHCl_3$).
Rf = 0,30 [chromatographie sur couche mince ; support : silice ; éluant : acétate d'éthyle].

**Stade C :** Sel de sodium de l'acide [[4-[17béta hydroxy 17alpha (3-hydroxy propyl) 3-oxo estra 4,9-dièn 11béta-yl] phényl] méthylamino] acétique.

Une solution de 1,5 cm3 de soude normale dans 4 cm3 de méthanol est dégazée sous barbotage d'azote et on y introduit 0,8 g du produit preparé au stade précédent. Après chauffage à 80°C pendant 1 heure, la solution est refroidie et chromatographiée sur une colonne Bondapack en éluant avec le mélange méthanol-eau (3-7). Les éluats sont lyophilisés; on obtient 0,637 g du produit recherché.
$[alpha]_D$ = +179° (c = 0,95% dans EtOH).

**Exemple 5 : Sel de sodium de l'acide [[4-[3-oxo spiro [estra 4,9-dièn 17béta, 2'(5'H) furan] 11béta-yl] phényl] méthylamino] acétique.**

**Stade A :** [[4-[3-oxo spiro [estra 4,9-dièn 17béta, 2'(5'H) furan] 11béta-yl] phényl] méthylamino] acétate d'éthyle.

2,08 g du produit préparé au stade B de l'exemple 3 sont dissous dans 40 cm3 de pyridine ; à la solution refroidie à 0°C, on ajoute, sous agitation 4 g de chlorure de tosyle, amène à la température ambiante et laisse réagir pendant 4 heures. Après dilution à l'eau en refroidissant et extraction au chlorure de méthylène, la phase organique est lavée à l'eau, séchée et évaporée sous pression réduite. Le produit brut ainsi récupéré est chromatographié sur silice en éluant avec un mélange cyclohexane-acétate d'éthyle (1-1). On obtient 1,74 g du produit recherché.
$[alpha]_D$ = +229° (c = 1% dans $CHCl_3$).
Rf = 0,38 [chromatographie sur couche mince ; support : silice ; éluant : mélange cyclohexane-acétate d'éthyle (6-4)].

**Stade B :** Sel de sodium de l'acide [[4-[3-oxo spiro [estra 4,9-dièn 17béta, 2'(5'H) furan] 11béta-yl] phényl] méthylamino] acétique.

1,01 g du produit préparé au stade précédent sont dissous dans 30 cm3 de méthanol et 2 cm3 de soude normale ; la solution ainsi formée est agitée sous azote pendant 15 heures et demie puis évaporée sous pression réduite sans chauffer. Le résidu obtenu est chromatographié sur Bondapack KC 18 [R] en éluant avec un mélange méthanol-eau (45-55). L'éluant est lyophilisé et on obtient 0,655 g du sel recherché.
$[alpha]_D$ = +253° (c = 1% dans EtOH).

**Exemple 6 : Sel de sodium de l'acide [[4-[4',5'-dihydro 3-oxo spiro [estra 4,9-dièn 17béta, 2'(3'H) furan] 11béta-yl] phényl] méthylamino] acétique.**

**Stade A :** [[4-[4',5'-dihydro 3-oxo spiro [estra 4,9-dièn 17béta, 2'(3'H) furan] 11béta-yl] phényl] méthylamino] acétate d'éthyle.

1,71 g du produit préparé au stade B de l'exemple 4 sont dissouts dans 34 cm3 de pyridine. Après refroidissement de la solution ainsi formée au bain de glace, 1,7 g de chlorure de tosyle sont ajoutés sous agitation puis le milieu ractionnel est amené à température ambiante et laissé 3 heures. Quelques morceaux de glace puis de l'eau sont alors ajoutés et le tout est extrait à l'acétate d'éthyle. La phase organique est lavée à l'eau, séchée et évaporée pour conduire à un résidu qui est chromatographié sur silice en éluant avec un mélange cyclohexane-acétate d'éthyle (7-3) et fournit ainsi 1,375 g du produit recherché.
$[alpha]_D$ = +174° (c = 1% dans $CHCl_3$).
Rf = 0,33 [chromatographie sur couche mince ; support : silice ; éluant : mélange cyclohexane-acétate d'éthyle (7-3)].

**Stade B :** Sel de sodium de l'acide [[4-[4′,5′-dihydro 3-oxo spiro [estra 4,9-dièn 17béta, 2′(3′H) furan] 11béta-yl] phényl] méthylamino] acétique.

0,92 g du produit préparé au stade précédent sont dissous dans 9 cm3 de méthanol et 1,8 cm3 de soude normale. Après 1 heure de chauffage au bain d'huile à 70 °C sous pression réduite, la solution est concentrée et chromatographiée sur Bondapack [R] (10 microns) en éluant avec les mélanges méthanol-eau (30-70) puis (50-50). L'éluant est lyophilisé et conduit à 0,65 g du sel recherché.
$[alpha]_D$ = +187 ° (c = 1% dans l'éthanol).

**Exemple 7 : Sel de sodium de l'acide [[4-[17béta hydroxy 3-oxo 17alpha (2-propynyl) estra 4,9-dièn 11béta-yl] phényl] méthylamino] acétique.**

**Stade A :** [[4-[17béta hydroxy 3-oxo 17alpha (2-propynyl) estra 4,9-dièn 11béta-yl] phényl] méthylamino] acétate d'éthyle.

1,178 g de 17béta hydroxy 11béta [4-(méthylamino) phényl] 17alpha (2-propynyl) estra 4,9-dièn-3-one sont dissous dans 41 cm3 de toluène avec 2,3 cm3 de triéthylamine. 2,3 cm3 de bromoacétate d'éthyle sont ensuite ajoutés et la solution est agitée pendant 1 heure et demie à 80 °C, refroidie puis diluée avec une solution aqueuse saturée de bicarbonate de sodium. La phase aqueuse est décantée et réextraite à l'acétate d'éthyle. La phase organique est lavée à l'eau, séchée sur sulfate de sodium et évaporée à sec sous pression réduite. Le résidu obtenu est chromatographié sur silice en éluant avec le mélange acétate d'éthyle-essence G (50-50) et conduit à 1,038 g de produit que l'on recristallise dans un mélange chlorure de méthylène-éther isopropylique pour obtenir 0,968 g correspondant au produit recherché. F = 195 °C.
Rf = 0,25 [chromatographie sur couche mince ; support : silice $F_{254}$ Mack 60 [R] ; éluant : mélange cyclohexane-acétate d'éthyle (7-3)].

**Stade B :** Sel de sodium de l'acide [[4-[17béta hydroxy 3-oxo 17alpha (2-propynyl) estra 4,9-dièn 11béta-yl] phényl] méthylamino] acétique.

0,894 g du produit préparé au stade précédent sont dissous dans 18 cm3 d'éthanol absolu avec 1,8 cm3 de soude normale. Après 9 heures d'agitation à température ambiante, 13 cm3 d'éthanol absolu sont ajoutés et l'agitation est poursuivie pendant 25 heures. La solution est ensuite filtrée et le filtrat évaporé à sec sous pression réduite. Le résidu obtenu est chromatographié sur Bondapack KC 18 [R] en éluant avec les mélanges méthanol-eau (30-70) puis (50-50). L'éluat est concentré sous pression réduite pour éliminer le méthanol, filtré sur membrane millipore 0,45 $\mu$ et lyophilisé. 0,704 g du produit recherché est ainsi recueilli.
$[alpha]_D$ = +192 ° ± 3 ° (c = 1% dans EtOH).
Rf = 0,73 [chromatographie sur couche mince ; support : KC 18 Whatman [R] ; éluant : mélange méthanol-eau (70-30)].

**Exemple 8 : Sel de sodium de l'acide [[4-[17béta hydroxy 3-oxo 17alpha (2- propényl) estra 4,9-dièn 11béta-yl] phényl] méthylamino] acétique.**

**Stade A :** [[4-[17béta hydroxy 3-oxo 17alpha (2-propényl) estra 4,9-dièn 11béta-yl] phényl] méthylamino] acétate d'éthyle.

La préparation du produit est effectuée à partir de 1,068 g de 17béta hydroxy 11béta [4-(méthylamino) phényl] 17alpha (2-propényl) estra 4,9-dièn-3-one dans 37 cm3 de toluène avec 2,1 cm3 de triéthylamine et 2,1 cm3 de bromoacétate d'éthyle, en opérant de la même manière qu'au stade A de l'exemple 7. Après recristallisation dans le mélange chlorure de méthylène-éther isopropylique, on obtient 1,151 g du produit recherché sous forme de cristaux blancs. F = 191 °C.
Rf = 0,27 [chromatographie sur couche mince ; support : silice $F_{254}$ Mack 60 [R] ; éluant : mélange acétate d'éthyle-essence G (50-50)].

**Stade B :** Sel de sodium de l'acide [[4-[17béta hydroxy 3-oxo 17alpha (2-propényl) estra 4,9-dièn 11béta-yl] phényl] méthylamino] acétique.

1,079 g du produit préparé au stade précédent sont dissous dans 22 cm3 d'éthanol et 2,17 cm3 de soude normale. Après 3 heures d'agitation à température ambiante, 3 cm3 d'éthanol sont ajoutés et l'agitation est poursuivie pendant 23 heures. La solution est alors filtrée et le filtrat évaporé sous pression réduite. Le résidu ainsi obtenu est chromatographié sur KC 18 Bondapack [R] en éluant avec les mélanges méthanol-eau (30-70) puis (50-50). L'éluat est concentré pour éliminer le méthanol, filtré et lyophilisé. On obtient 0,878 g du sel recherché.
$[alpha]_D$ = +203° ± 3° (c = 1% dans EtOH).
Rf = 0,63 [chromatographie sur couche mince ; support KC 18 Whatman [R] ; éluant : mélange méthanol-eau (7-3)].

**Exemple 9 : Sel de sodium de l'acide [[4-[21-chloro 17béta hydroxy 3-oxo 19-nor 17alpha pregna 4,9-dièn 20-yn 11béta-yl] phényl] méthylamino] acétique.**

**Stade A :** [[4-[21-chloro 17béta hydroxy 3-oxo 19-nor 17alpha pregna 4,9- dièn 20-yn 11béta-yl] phényl] méthylamino] acétique.

1,38 g de 21-chloro 17béta hydroxy 11béta [4-(méthylamino) phényl] 19-nor 17alpha pregna 4,9-dièn 20-yn 3-one sont dissous dans 47 cm3 de toluène avec 2,5 cm3 de triéthylamine. Après addition de 2,5 cm3 de bromoacétate d'éthyle, la solution est agitée pendant 2 heures à 80°C, puis refroidie et ajoutée à une solution aqueuse saturée de bicarbonate de sodium. La phase aqueuse est décantée et la phase organique est lavée à l'eau, séchée sur sulfate de sodium et évaporée à sec sous pression réduite. Le produit brut obtenu est chromatographié sur silice en éluant avec le mélange acétate d'éthyle-essence G (40-60) et conduit après recristallisation dans le mélange chlorure de méthylène-éther isopropylique, à 1,555 g de l'ester souhaité. F = 105°C.
Rf = 0,27 [chromatographie sur couche mince ; support : silice $F_{254}$ Mack 60 [R] ; éluant : mélange acétate d'éthyle-essence G (1-1)].

**Stade B :** Sel de sodium de l'acide [[4-[21-chloro 17béta hydroxy 3-oxo 19-nor 17alpha pregna 4,9-dièn 20-yn 11béta-yl] phényl] méthylamino] acétique.

1,494 g du produit préparé au stade précédent sont dissous dans 29 cm3 d'éthanol absolu et 2,9 cm3 de soude normale. La solution est agitée pendant 22 heures et 30 minutes à température ambiante, filtrée et le filtrat évaporé à sec. Le résidu obtenu est chromatographié sur KC 18 Bondapack [R] en éluant avec les mélanges méthanol-eau (30-70) puis (50-50). L'éluat est concentré pour éliminer le méthanol, filtré et lyophilisé. On obtient ainsi 1,23 g du sel souhaité. $[alpha]_D$ = 124° ± 2,5° (c = 1% dans EtOH).
Rf 0,67 [chromatographie sur couche mince ; support : KC 18 Whatman [R] ; éluant : mélange méthanol-eau (70-30)].

**Exemple 10 :**

On a préparé le collyre répondant à la formule suivante :

| | |
|---|---|
| Produit de l'exemple 1 | 2 g |
| Excipient | 100 ml |

(Détail de l'excipient : eau distillée, chlorure de sodium, méthylcellulose, borate de sodium).

## ETUDE PHARMACOLOGIQUE

### 1) Mesure de l'affinité relative de liaison pour les récepteurs des hormones stéroïdiennes :

Récepteur glucocorticoïde du thymus de rat :

Des rats mâles de 160 à 200 g sont surrénalectomisés, 4 à 8 jours après cette ablation, les animaux sont sacrifiés et les thymus sont prélevés et homogénéisés à 0 °C à l'aide d'un Potter téflon-verre dans un tampon (TSD) Tris 10mM, saccharose 0,25M, dithiothreitol 2mM, HCl pH 7,4 (1 g de tissu pour 10 ml de TSD). L'homogénat est ensuite ultracentrifugé (105000 g x 90mn) à 0 °C. Des aliquotes du surnageant ainsi obtenu "cytosol" sont incubées à 0 °C pendant 4 heures et 24 heures avec une concentration constante (T = 2,5 nM) de dexaméthasone tritiée en présence de concentrations croissantes (0 - 2.500 nM) soit de dexaméthasone froide ou du produit froid à tester. La concentration de la dexaméthasone tritiée liée (B) est ensuite mesurée dans chaque incubat par la technique d'adsorption au charbon-destran.

Récepteur progestérone de l'utérus de lapin :

Des lapines impubères d'environ 1 kg reçoivent une application cutanée de 25 µg d'estradiol. Cinq jours après ce traitement, les animaux sont sacrifiés, les utérus sont prèlevés, pesés et homogénéisés à 0 °C à l'aide d'un Potter téflon-verre dans un tampon (TS) Tris 10 mM, saccharose 0,25M, HCl pH 7,4 (1 g de tissu pour 50 ml de TS). L'homogénat est ensuite ultra-centrifugé (105000 g x 90 mn) à 0 °C. Des aliquotes de surnageant ainsi obtenu "cytosol" sont incubées à 0 °C pendant 2 heures et 24 heures avec une concentration constante (T = 5 nm) de R 5020 tritié (17,21-diméthyl 19-nor 4,9pregnadièn-3,20-dione, progestomimétique puissant très affiné pour le récepteur de la progestérone, en présence de concentrations croissantes (0 - 2500 nM) de progestérone froide, ou du produit à tester.

La concentration de R 5020 tritié lié (B) est ensuite mesurée dans chaque incubat par la technique d'adsorption au charbon-dextran.

Récepteur androgène de la prostate de rat :

Des rats mâles de 160 à 200 g sont castrés. 24 heures après la castration, les animaux sont sacrifiés, les prostates sont prélevées, pesées et homogénéisés à 0 °C, à l'aide d'un Potter téflon-verre dans un tampon (TSDPM) Tris 10 mM, saccharose 0,25M, phénylméthanesulfonylfluoride 0,1 nM, molybdate 20 mM, dithiotreitol 2 mM, HCl pH 7,4 (1 g de tissu pour 5 ml de TSDPM). L'homogénat est ensuite ultra-centrifugé (105000 g x 60 mn) à 0 °C. Des aliquotes du surnageant ainsi obtenu "cytosol" sont incubées à 0 °C avec une concentration constante de testostérone tritiée en présence de concentrations croissantes (0 - 1000 nM) de testostérone froide ou du produit à tester. Après 1/2 heure et 24 heures d'incubation, la concentration de testostérone tritiée liée (B) est mesurée dans chaque incubat par la technique d'adsorption au charbon-dextran.

Calcul de l'affinité relative de liaison :

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du lagarithme de la concentration du produit froid testé.

On détermine la droite d'équation

$$I_{50} = \left(\frac{B}{T} max + \frac{B}{T} min\right)/2.$$

$\frac{B}{T}$ max = Pourcentage de l'hormone tritiée liée pour une incubation de cette hormone tritiée à la concentration (T).

$\frac{B}{T}$ min = Pourcentage de l'hormone tritiée liée pour une incubation de hormone tritiée à la concentration (T) en présence d'un grand excès d'hormone froide ($2500.10^{-9}$ M).

Les intersections de la droite $I_{50}$ et des courbes permettent d'évaluer les concentrations de l'hormone de référence froide (CH) et du produit froid testé (CX) qui inhibent de 50% la liaison de l'hormone tritiée sur le récepteur.

L'affinité relative de liaison (ARL) du produit testé est déterminée par l'équation

$$ARL = 100 \frac{(CH)}{(CX)}$$

Les résultats sont les suivants :

| Exemples | Progestérone | | Glucocorticoïde | | Androgène | |
|---|---|---|---|---|---|---|
| | 2H | 24H | 4H | 24H | 0,5H | 5H |
| 1 | 22 | 107 | 101 | 129 | 2,1 | 10,1 |

**2) Activité anti-glucocorticoïde vis-à-vis de la dexaméthasone.**

Etude in vitro

Incorporation d'uridine dans les thymocytes de rat

Les glucocorticoïdes provoquent au niveau du tissu lymphoïde une inhibition de l'incorporation des nucléosides. La mesure de l'incorporation d'uridine radioactive dans les thymocytes en présence d'un produit à tester permet d'évaluer son activité glucocorticoïde.

Méthode

Elle s'inspire de la technique décrite par Dausse et Coll (3). Le thymus d'un rat surrénalectomisé (160 à 180 g) est prélevé, émincé et homogénéisé lentement à l'aide d'un homogénéiseur téflon-verre dans une solution de Hanks. La suspension cellulaire obtenue est filtrée sur gaze, puis centrifugée à 800 g x 10 mn. On procède ensuite à une nouvelle centrifugation à 800 g x 10 mn. Le culot ainsi obtenu est remis en suspension dans un milieu nutritif (M.E.M. Gibco) et la concentration cellulaire est ajustée à environ $20.10^6$ cellules par ml. Des aliquotes de 250 µl sont alors incubées sous carbogène pendant 3 heures à 37 °C avec $5.10^{-8}$ M de dexaméthasone en présence ou non de concentrations croissantes de produit ($10^{-8}$ M à $10^{-6}$ M).

On ajoute ensuite dans chaque incubat 0,1 µCi d'uridine tritiée et on continue l'incubation pendant 1 heure. Les incubats sont ensuite refroidis et additionnés de 1 ml d'une solution froide d'acide trichloracétique (TCA) à 5% poids/volume. Les précipités sont recueillis sur des filtres Whatman GF/C et sont lavés par 4 x 2 l de TCA à 5% glacé. La radioactivité retenue sur les filtres (représentant l'uridine tritiée incorporée dans les thymocytes) est mesurée à l'aide d'un spectomètre à scintillation liquide.

| Thymocytes % de l'inhibition de l'incorporation d'uridine Concentration molaire du produit à tester | | | |
|---|---|---|---|
| Produit de l'exemple | $10^{-6}$ M | $10^{-7}$ M | $10^{-8}$ M |
| 1 | 82 | 42 | 3 |
| 3 | 37 | 0 | 0 |

Les mesures précédentes que le produit de l'exemple 1 présente une bonne activité anti-glucocorticoïde.

## Revendications

**Revendications pour les Etats contractants suivants : AT, BE, CH, DE, DK, FR, GB, IT, LI, LU, NL, SE**

**1.** Les produits de formule générale (I) :

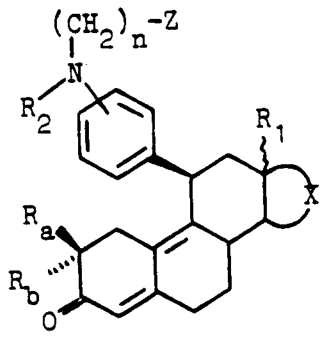

(I)

dans laquelle $R_1$ représente un radical hydrocarboné aliphatique renfermant de 1 à 8 atomes de carbone, $R_a$ et $R_b$ identiques ou différents représentent un atome d'hydrogène, un radical alkyle renfermant de 1 à 4 atomes de carbone, $R_2$ représente un atome d'hydrogène ou un radical alkyle renfermant de 1 à 12 atomes de carbone et éventuellement substitué, n représente une des valeurs de 1 à 6, Z représente un groupe carboxy libre ou salifié sous forme d'un sel de métal alcalin ou alcalino-terreux, d'un sel d'ammonium ou d'un sel d'amine, X représente le reste d'un cycle pentagonal ou hexagonal éventuellement substitué et éventuellement porteur d'une insaturation, et le trait ondulé en position 13 signifie que $R_1$ peut se trouver en position alpha ou béta.

**2.** Les produits de formule générale (I) telle que définie à la revendication 1 et répondant à la formule (I') :

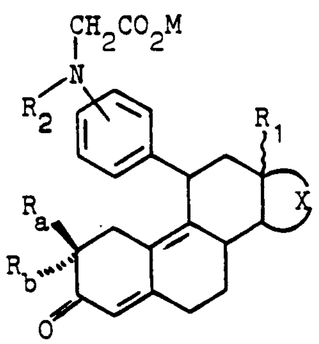

(I')

dans laquelle M représente un atome d'hydrogène ou un atome de sodium.

**3.** Les produits de formule générale (I) telle que définie à la revendication 1 ou 2, dans laquelle X représente un reste de formule

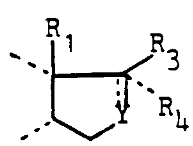

dans lequel $R_1$ conserve la même signification que précédemment, le trait pointillé en 16-17 symbolise la présence éventuelle d'une double liaison, Y représente un radical

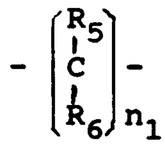

dans lequel $n_1$ représente le nombre 1 ou 2,

$R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$ identique ou différent de $R_5$ peut prendre l'une des valeurs indiquées pour $R_5$ et peut également représenter un radical hydroxyle, $R_3$ et $R_4$, identiques ou différents, représentent

soit un atome d'hydrogène,

soit un radical choisi dans le groupe formé par les radicaux OH, $Oalc_1$, $O\text{-}CO\text{-}alc_2$ dans lesquels $alc_1$ et $alc_2$ représentent un radical alkyle renfermant de 1 à 8 atomes de carbone ou aralkyle renfermant de 7 à 15 atomes de carbone,

soit un radical alkyle, alkényle ou alkynyle renfermant de 2 à 8 atomes de carbone et éventuellement substitué,

soit un radical

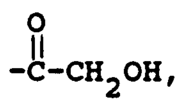

soit un radical $-COCH_2OCOalc_3$ dans lequel $alc_3$ représente un radical alkyle renfermant de 1 à 8 atomes de carbone éventuellement substitué ou un radical aralkyle renfermant de 7 à 15 atomes de carbone,

soit un radical $CO\text{-}CO_2H$,

soit un radical $CO\text{-}CO\text{-}alc_4$ dans lequel $alc_4$ représente un radical alkyle renfermant de 1 à 8 atomes de carbone,

soit un radical

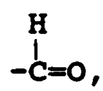

soit un radical

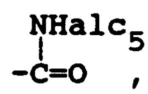

dans lequel $alc_5$ représente un radical alkyle renfermant de 1 à 8 atomes de carbone ou un radical aralkyle renfermant de 7 à 15 atomes de carbone,

soit un radical $-C{\equiv}N$,

soit $R_3$ et $R_4$ forment ensemble avec l'atome auxquels ils sont liés un radical

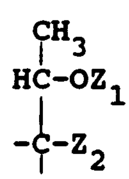

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone,

soit $R_3$ et $R_4$ forment ensemble avec l'atome auquel ils sont liés, ou bien un radical

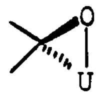

dans lequel U représente un radical bivalent $-(CH_2)n_2$ dans lequel $n_2$ représente un entier égal à 1, 2, 3 ou 4 ou un radical bivalent $-CH=CH-(CH_2)n_3-$ dans lequel $n_3$ représente un entier égal à 1 ou 2, ou bien un radical

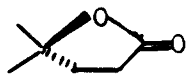

4. Les produits de formule générale (I) telle que définie à l'une quelconque des revendications 1 à 3, dans laquelle le cycle D du noyau stéroïde ne porte pas d'insaturation, $R_5$ et $R_6$ représentent un atome d'hydrogène et $n_1$ est égale à 1.

5. Les produits de formule générale (I) telle que définie à l'une quelconque des revendications 1 à 4, répondant à la formule (I″) :

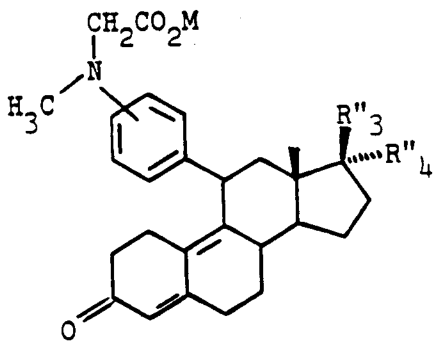

(I")

dans laquelle M est tel que défini précédemment et soit $R''_3$ représente un radical hydroxy et dans ce cas $R''_4$ représente ou bien un groupe

$$-C \equiv C-R_7$$

dans lequel les traits pointillés indiquent la présence éventuelle d'une deuxième ou d'une troisième liaison et $R_7$ représente un atome d'hydrogène, un atome d'halogène, un radical triméthylsilyle ou un radical méthyle éventuellement substitué par un ou plusieurs atomes d'halogène, un radical hydroxy ou par un groupe alkyloxy, alkylthio ou alkylamino renfermant de 1 à 4 atomes de carbone, dialkylamino renfermant de 2 à 8 atomes de carbone ou trialkylsilyle renfermant de 3 à 12 atomes de carbone ou bien un radical 2-propynyle ou 2-propényle, soit $R''_3$ et $R''_4$ représentent ensemble avec l'atome auquel ils sont liés un radical

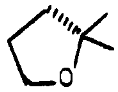

ou

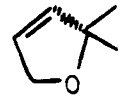

**6.** Le produit de formule générale (I) telle que définie à la revendication 4, dont le nom suit :
- sel de sodium de l'acide [[4-[17béta hydroxy 3-oxo 17alpha (1-propynyl) estra 4,9-dièn 11-béta-yl] phényl] méthylamino] acétique.

**7.** Procédé de préparation des produits de formule (I) telle que définie à la revendication 1, caractérisé en ce que les produits de formule ($II_a$) et ($II_b$) :

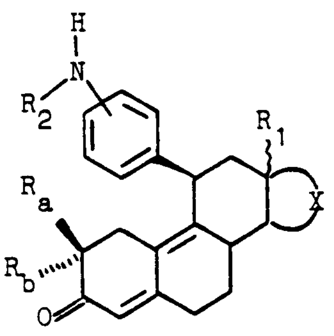

($II_a$)

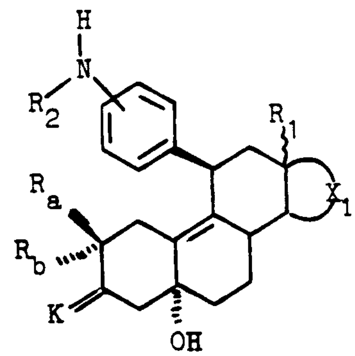

($II_b$)

dans lesquels $R_1$, $R_2$, $R_a$, $R_b$ et X sont tels que définis précédemment, $X_1$ a la signification indiquée à la revendication 1 pour X ainsi que ces valeurs dans lesquelles les fonctions réactives sont protégées et K représente une fonction oxo protégée, sont soumis en présence d'une base à l'action d'un halogénoester de formule (III) :

$Hal-(CH_2)n-COOR_7$ (III)

dans laquelle Hal représente un atome d'halogène, n a la signification indiquée précédemment et $R_7$ représente un radical alkyle renfermant de 1 à 4 atomes de carbone et éventuellement substitué par un ou plusieurs radicaux phényle, pour obtenir respectivement les produits de fomules ($IV_a$) et ($IV_b$) :

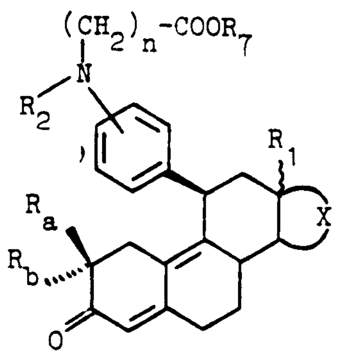

($IV_a$)

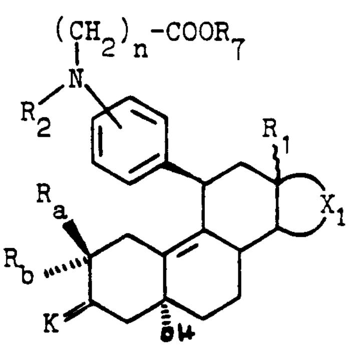

($IV_b$)

produits de formule ($IV_b$) que l'on soumet à une réaction de déshydratation et de déprotection éventuelle des fonctions réactives protégées pour obtenir les produits de formule ($IV_a$) et produits de formule ($IV_a$) que l'on soumet à un traitement basique puis, si désiré, acide pour obtenir les produits de formule (I).

**8.** Procédé selon la revendication 7, caractérisé en ce que les produits de formules ($IV_a$) et ($IV_b$) sont soumis, si désiré, à l'une ou plusieurs des réactions suivantes dans un ordre quelconque :

a) hydrogénation partielle ou totale des liaisons multiples que peuvent comporter X et $X_a$,

b) cyclisation de deux fonctions réactives portées par X de manière à former un cycle spirannique en position 17 du noyau stéroïde.

**9.** A titre de médicaments, les produits de formule (I) telle que définie à la revendication 1.

**10.** A titre de médicaments, les produits de formule (I) telle que définie à l'une des revendications 2 à 6.

**11.** Compositions pharmaceutiques renfermant comme principe actif l'un au moins des médicaments définis selon les revendications 9 ou 10.

**12.** A titre de produits industriels nouveaux, les produits de formules ($IV_a$) et ($IV_b$) telles que définies précédemment.

**Revendications pour l'Etat contractant suivant : ES**

**1.** Procédé pour préparer les produits de formule générale (I) :

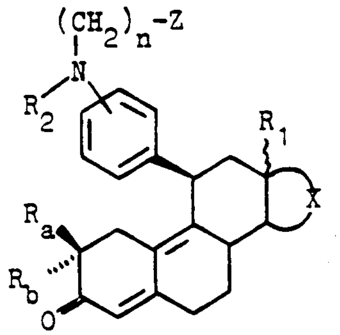

(I)

dans laquelle $R_1$ représente un radical hydrocarboné aliphatique renfermant de 1 à 8 atomes de carbone, $R_a$ et $R_b$ identiques ou différents représentent un atome d'hydrogène, un radical alkyle renfermant de 1 à 4 atomes de carbone, $R_2$ représente un atome d'hydrogène ou un radical alkyle renfermant de 1 à 12 atomes de carbone et éventuellement substitué, n représente une des valeurs de 1 à 6, Z représente un groupe carboxy libre ou salifié sous forme d'un sel de métal alcalin ou alcalino-terreux, d'un sel d'ammonium ou d'un sel d'amine, X représente le reste d'un cycle pentagonal ou hexagonal éventuellement substitué et éventuellement porteur d'une insaturation, et le trait ondulé en position 13 signifie que $R_1$ peut se trouver en position alpha ou béta, caractérisé en ce que les produits de formule ($II_a$) et ($II_b$) :

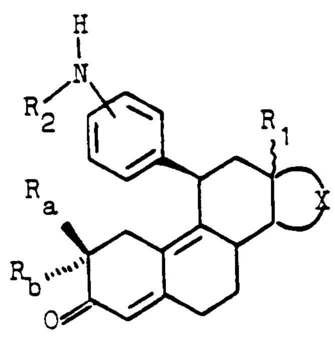

($II_a$)

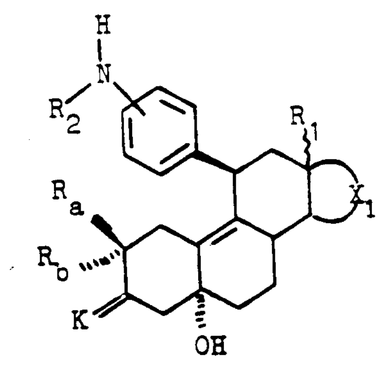

($II_b$)

dans lesquels $R_1$, $R_2$, $R_a$, $R_b$ et X sont tels que définis précédemment, $X_1$ a la signification indiquée à la revendication 1 pour X ainsi que ces valeurs dans lesquelles les fonctions réactives sont protégées et K représente une fonction oxo protégée, sont soumis en présence d'une base à l'action d'un

halogénoester de formule (III) :

$Hal-(CH_2)n-COOR_7$ (III)

dans laquelle Hal représente un atome d'halogène, n a la signification indiquée précédemment et $R_7$ représente un radical alkyle renfermant de 1 à 4 atomes de carbone et éventuellement substitué par un ou plusieurs radicaux phényle, pour obtenir respectivement les produits de fomules ($IV_a$) et ($IV_b$) :

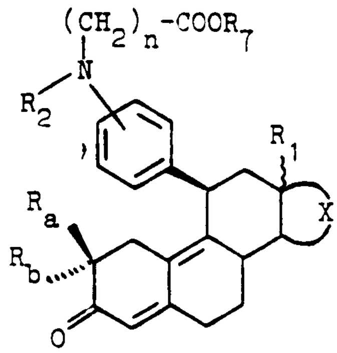

($IV_a$)

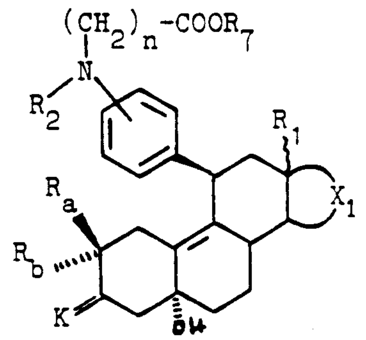

($IV_b$)

produits de formule ($IV_b$) que l'on soumet à une réaction de déshydratation et de déprotection éventuelle des fonctions réactives protégées pour obtenir les produits de formule ($IV_a$) et produits de formule ($IV_a$) que l'on soumet à un traitement basique puis, si désiré, acide pour obtenir les produits de formule (I).

**2.** Procédé selon la revendication 1, caractérisé en ce que les produits de formules ($IV_a$) et ($IV_b$) sont soumis, si désiré, à l'une ou plusieurs des réactions suivantes dans un ordre quelconque :
a) hydrogénation partielle ou totale des liaisons multiples que peuvent comporter X et $X_a$,
b) cyclisation de deux fonctions réactives portées par X de manière à former un cycle spirannique en position 17 du noyau stéroïde.

**3.** Procédé selon la revendication 1 ou 2 pour la préparation des produits de formule (I) telle que définie à la revendication 1, répondant à la formule (I') :

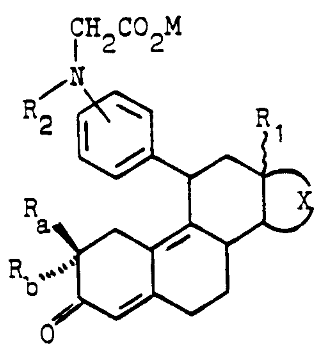

(I')

dans laquelle M représente un atome d'hydrogène ou un atome de sodium, caractérisé en ce que l'on utilise au départ un produit de formule (III) dans laquelle n = 1.

**4.** Procédé selon l'une quelconque des revendication 1 à 3, caractérisé en ce que l'on utilise au départ un produit de formule ($II_a$) ou ($II_b$), dans laquelle X représente un reste de formule

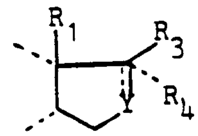

dans lequel $R_1$ conserve la même signification que précédemment, le trait pointillé en 16-17 symbolise la présence éventuelle d'une double liaison, Y représente un radical

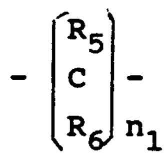

dans lequel $n_1$ représente le nombre 1 ou 2,
$R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$ identique ou différent de $R_5$ peut prendre l'une des valeurs indiquées pour $R_5$ et peut également représenter un radical hydroxyle, $R_3$ et $R_4$, identiques ou différents, représentent
soit un atome d'hydrogène,
soit un radical choisi dans le groupe formé par les radicaux OH, $Oalc_1$, $O\text{-}CO\text{-}alc_2$ dans lesquels $alc_1$ et $alc_2$ représentent un radical alkyle renfermant de 1 à 8 atomes de carbone ou aralkyle renfermant de 7 à 15 atomes de carbone,
soit un radical alkyle, alkényle ou alkynyle renfermant de 2 à 8 atomes de carbone et éventuellement substitué,
soit un radical

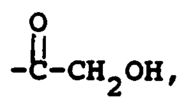

soit un radical $-COCH_2OCOalc_3$ dans lequel $alc_3$ représente un radical alkyle renfermant de 1 à 8 atomes de carbone éventuellement substitué ou un radical aralkyle renfermant de 7 à 15 atomes de carbone,
soit un radical $CO\text{-}CO_2H$,
soit un radical $CO\text{-}CO\text{-}alc_4$ dans lequel $alc_4$ représente un radical alkyle renfermant de 1 à 8 atomes de carbone,
soit un radical

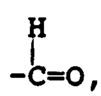

soit un radical

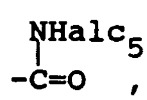

dans lequel $alc_5$ représente un radical alkyle renfermant de 1 à 8 atomes de carbone ou un radical aralkyle renfermant de 7 à 15 atomes de carbone,
soit un radical $-C{\equiv}N$,
soit $R_3$ et $R_4$ forment ensemble avec l'atome auxquels ils sont liés un radical

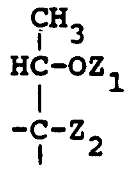

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone, soit $R_3$ et $R_4$ forment ensemble avec l'atome auquel ils sont liés, ou bien un radical

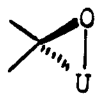

dans lequel U représente un radical bivalent $-(CH_2)n_2$ dans lequel $n_2$ représente un entier égal à 1, 2, 3 ou 4 ou un radical bivalent $-CH=CH-(CH_2)n_3-$ dans lequel $n_3$ représente un entier égal à 1 ou 2, ou bien un radical

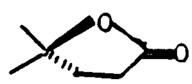

5. Procédé selon la revendication 4, caractérisé en ce l'on utilise au départ un produit de formule ($II_a$) ou ($II_b$), dans laquelle le cycle D du noyau stéroïde ne porte pas d'insaturation, $R_5$ et $R_6$ représentent un atome d'hydrogène et $n_1$ est égale à 1.

6. Procédé selon l'une quelconque des revendications 1 à 5 pour la préparation des produits de formule (I) telle que définie à la revendication 1, répondant à la formule (I") :

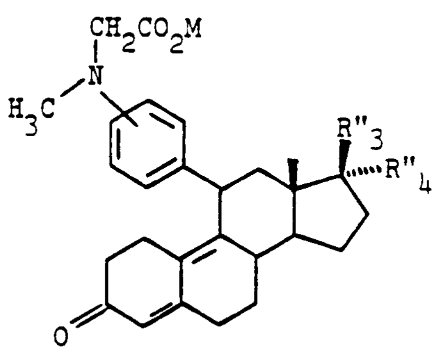

(I")

dans laquelle M est tel que défini précédemment et soit $R''_3$ représente un radical hydroxy et dans ce cas $R''_4$ représente ou bien un groupe $-C$ $C-R_7$ dans lequel les traits pointillés indiquent la présence éventuelle d'une deuxième ou d'une troisième liaison et $R_7$ représente un atome d'hydrogène, un atome d'halogène, un radical triméthylsilyle ou un radical méthyle éventuellement substitué par un ou plusieurs atomes d'halogène, un radical hydroxy ou par un groupe alkyloxy, alkylthio ou alkylamino renfermant de 1 à 4 atomes de carbone, dialkylamino renfermant de 2 à 8 atomes de carbone ou trialkylsilyle renfermant de 3 à 12 atomes de carbone ou bien un radical 2-propynyle ou 2-propényle, soit $R''_3$ et $R''_4$ représentent ensemble

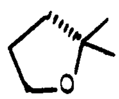

ou

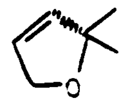

avec l'atome auquel ils sont liés un radical caractérisé en ce que des produits de formule ($II_a$) ou ($II_b$) telle que définie à la revendication 1, répondant à la formule ($II''_a$) et ($II''_b$) :

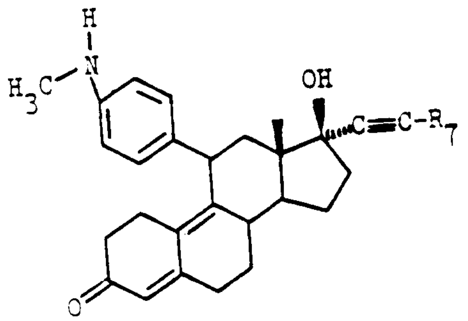

**($II''_a$)**

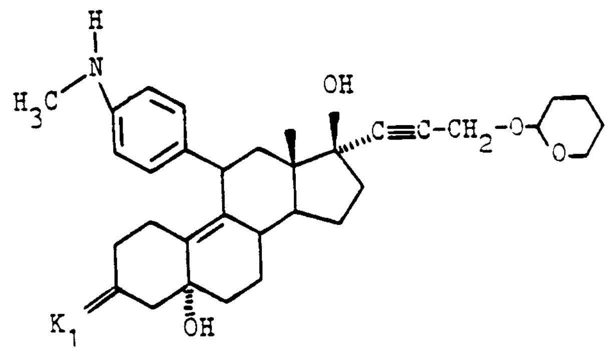

**($II''_b$)**

dans laquelle $K_1$ représente un groupe oxo protégé sous forme d'un radical éthylénedioxy ou d'un acétal diméthylique sont soumis à l'action du bromoacétate d'éthyle en présence d'une base azotée pour obtenir respectivement les produits de formules ($IV''_a$) et ($IV''_b$) :

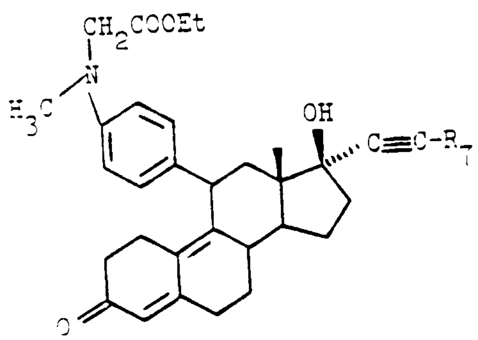

**($IV''_a$)**

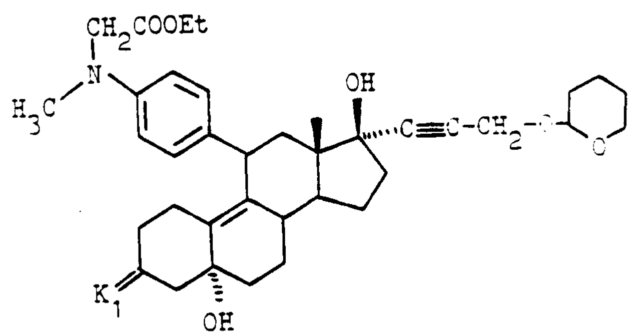

**($IV''_b$)**

produits de formules ($IV''_a$) et ($IV''_B$) qui si désiré sont hydrogénés pour obtenir respectivement suivant le catalyseur utilisé soit les produits de formules ($V''_a$) et ($V''_b$) :

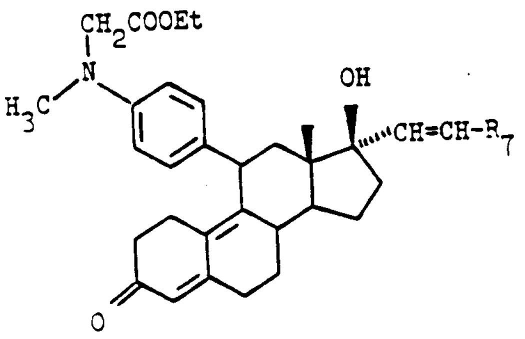

(V"$_a$)

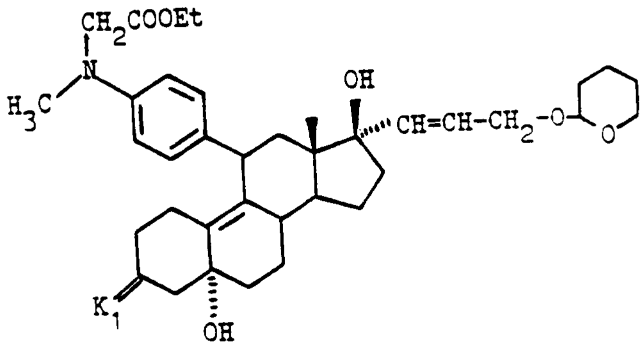

(V"$_b$

soit les produits de formule (VI"$_a$) et (VI"$_b$) :

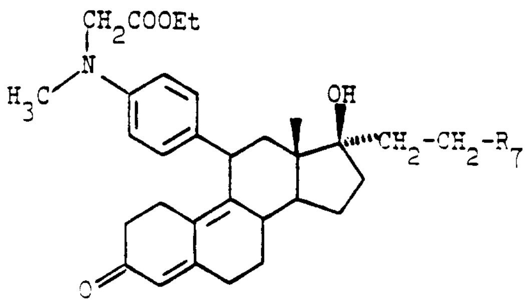

(VI"$_a$)

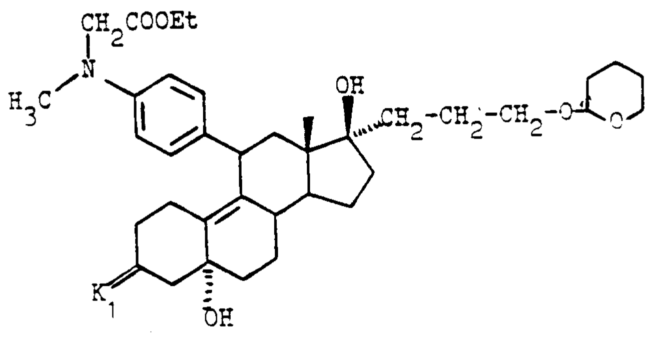

(VI"$_b$)

produits de formule (V"$_b$) et (VI"$_b$) qui par traitement acide conduisent aux produits de formules (V"$_c$) et (VI"$_c$) :

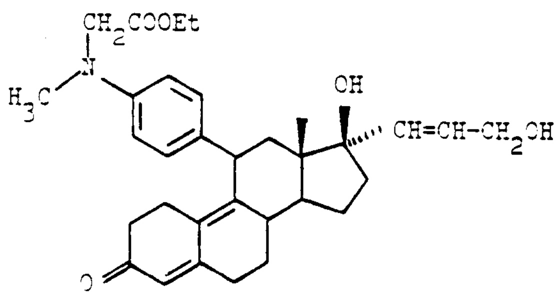

(V"$_c$)

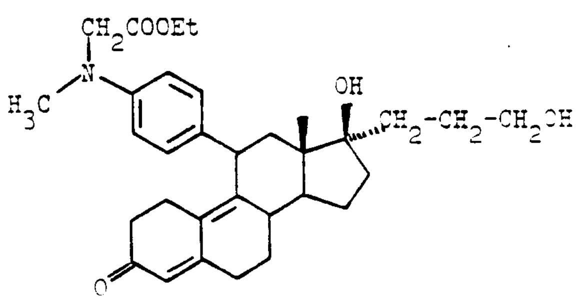

(VI"$_c$)

produits de formules (V"$_c$) et (VI"$_c$) que si désiré l'on fait réagir avec du chlorure de tosyle dans la pyridine pour obtenir respectivement les produits de formules (V"$_d$) et (VI"$_d$) :

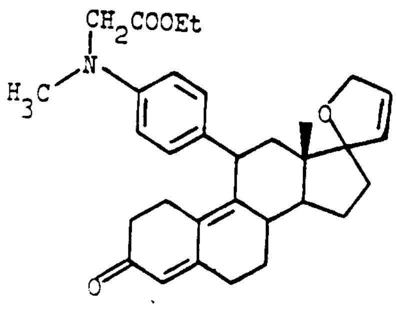

(V"$_d$)

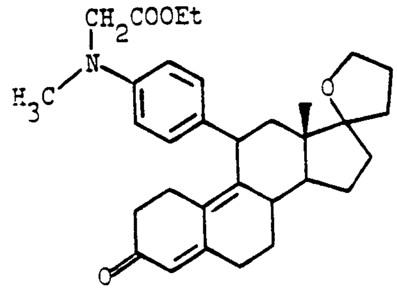

(VI"$_d$)

produits de formules (IV"$_a$), (V"$_a$), (V"$_c$), VI"$_a$), (VI"$_c$, (V"$_d$) et (VI"$_d$) que l'on traite avec une solution hydroalcoolique de soude puis si désiré par l'acide chlorhydrique pour obtenir les produits de formule (I").

**Revendications pour l'Etat contractant suivant : GR**

1. Procédé pour préparer les produits de formule générale (I) :

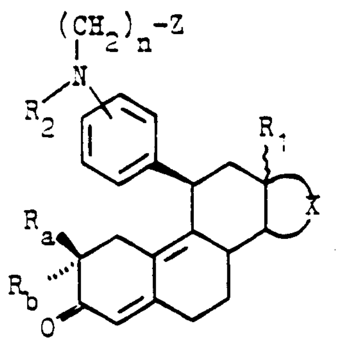

(I)

dans laquelle $R_1$ représente un radical hydrocarboné aliphatique renfermant de 1 à 8 atomes de carbone, $R_a$ et $R_b$ identiques ou différents représentent un atome d'hydrogène, un radical alkyle renfermant de 1 à 4 atomes de carbone, $R_2$ représente un atome d'hydrogène ou un radical alkyle renfermant de 1 à 12 atomes de carbone et éventuellement substitué, n représente une des valeurs de 1 à 6, Z représente un groupe carboxy libre ou salifié sous forme d'un sel de métal alcalin ou alcalino-terreux, d'un sel d'ammonium ou d'un sel d'amine, X représente le reste d'un cycle pentagonal ou hexagonal éventuellement substitué et éventuellement porteur d'une insaturation, et le trait ondulé en position 13 signifie que $R_1$ peut se trouver en position alpha ou béta, caractérisé en ce que les produits de formule ($II_a$) et ($II_b$) :

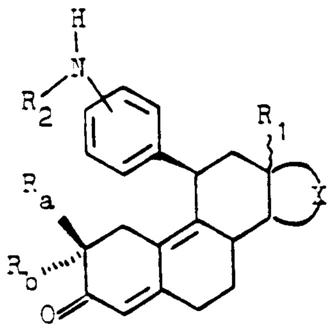

$(II_a)$

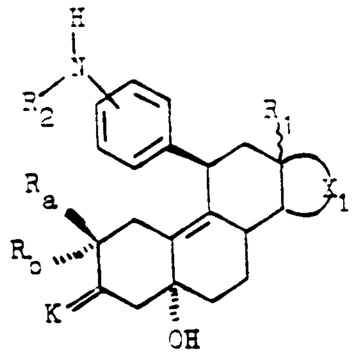

$(II_b)$

dans lesquels $R_1$, $R_2$, $R_a$, $R_b$ et X sont tels que définis précédemment, $X_1$ a la signification indiquée à la revendication 1 pour X ainsi que ces valeurs dans lesquelles les fonctions réactives sont protégées et K représente une fonction oxo protégée, sont soumis en présence d'une base à l'action d'un halogénoester de formule (III) :

$Hal\text{-}(CH_2)n\text{-}COOR_7$ (III)

dans laquelle Hal représente un atome d'halogène, n a la signification indiquée précédemment et $R_7$ représente un radical alkyle renfermant de 1 à 4 atomes de carbone et éventuellement substitué par un ou plusieurs radicaux phényle, pour obtenir respectivement les produits de fomules $(IV_a)$ et $(IV_b)$ :

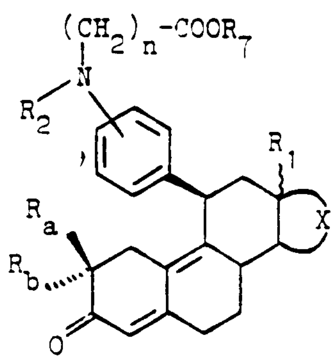

$(IV_a)$

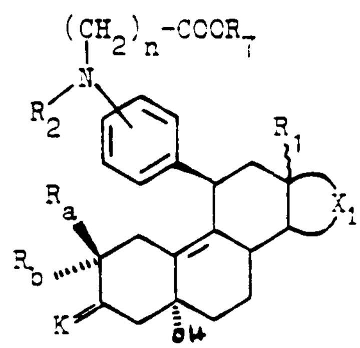

$(IV_b)$

produits de formule $(IV_b)$ que l'on soumet à une réaction de déshydratation et de déprotection éventuelle des fonctions réactives protégées pour obtenir les produits de formule $(IV_a)$ et produits de formule $(IV_a)$ que l'on soumet à un traitement basique puis, si désiré, acide pour obtenir les produits de formule (I).

**2.** Procédé selon la revendication 1, caractérisé en ce que les produits de formules $(IV_a)$ et $(IV_b)$ sont soumis, si désiré, à l'une ou plusieurs des réactions suivantes dans un ordre quelconque :

a) hydrogénation partielle ou totale des liaisons multiples que peuvent comporter X et $X_a$,

b) cyclisation de deux fonctions réactives portées par X de manière à former un cycle spirannique en position 17 du noyau stéroïde.

**3.** Procédé selon la revendication 1 ou 2 pour la préparation des produits de formule (I) telle que définie à la revendication 1, répondant à la formule (I') :

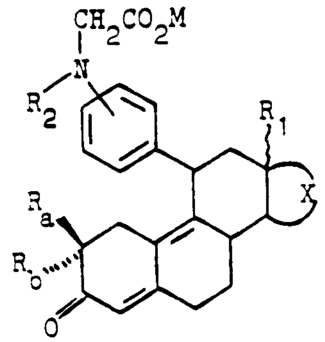

(I')

dans laquelle M représente un atome d'hydrogène ou un atome de sodium, caractérisé en ce que l'on utilise au départ un produit de formule (III) dans laquelle n = 1.

**4.** Procédé selon l'une quelconque des revendication 1 à 3, caractérisé en ce que l'on utilise au départ un produit de formule ($II_a$) ou ($II_b$), dans laquelle X représente un reste de formule

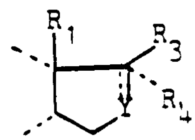

dans lequel $R_1$ conserve la même signification que précédemment, le trait pointillé en 16-17 symbolise la présence éventuelle d'une double liaison, Y représente un radical

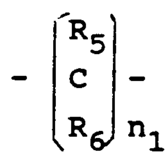

dans lequel $n_1$ représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$ identique ou différent de $R_5$ peut prendre l'une des valeurs indiquées pour $R_5$ et peut également représenter un radical hydroxyle, $R_3$ et $R_4$, identiques ou différents, représentent
soit un atome d'hydrogène,
soit un radical choisi dans le groupe formé par les radicaux OH, $Oalc_1$, O-CO-$alc_2$ dans lesquels $alc_1$ et $alc_2$ représentent un radical alkyle renfermant de 1 à 8 atomes de carbone ou aralkyle renfermant de 7 à 15 atomes de carbone,
soit un radical alkyle, alkényle ou alkynyle renfermant de 2 à 8 atomes de carbone et éventuellement substitué,
soit un radical

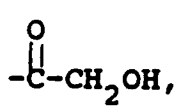

soit un radical $-COCH_2OCOalc_3$ dans lequel $alc_3$ représente un radical alkyle renfermant de 1 à 8 atomes de carbone éventuellement substitué ou un radical aralkyle renfermant de 7 à 15 atomes de carbone,
soit un radical $CO-CO_2H$,
soit un radical CO-CO-$alc_4$ dans lequel $alc_4$ représente un radical alkyle renfermant de 1 à 8 atomes de carbone,

soit un radical

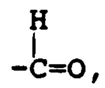

soit un radical

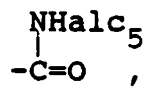

dans lequel $alc_5$ représente un radical alkyle renfermant de 1 à 8 atomes de carbone ou un radical aralkyle renfermant de 7 à 15 atomes de carbone,
soit un radical -C≡N,
soit $R_3$ et $R_4$ forment ensemble avec l'atome auxquels ils sont liés un radical

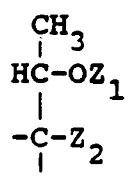

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone,
soit $R_3$ et $R_4$ forment ensemble avec l'atome auquel ils sont liés,
ou bien un radical

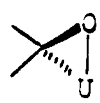

dans lequel U représente un radical bivalent $-(CH_2)n_2$ dans lequel $n_2$ représente un entier égal à 1, 2, 3 ou 4 ou un radical bivalent $-CH=CH-(CH_2)n_3-$ dans lequel $n_3$ représente un entier égal à 1 ou 2,
ou bien un radical

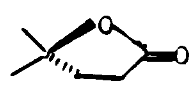

**5.** Procédé selon la revendication 4, caractérisé en ce l'on utilise au départ un produit de formule ($II_a$) ou ($II_b$), dans laquelle le cycle D du noyau stéroïde ne porte pas d'insaturation, $R_5$ et $R_6$ représentent un atome d'hydrogène et $n_1$ est égale à 1.

**6.** Procédé selon l'une quelconque des revendications 1 à 5 pour la préparation des produits de formule (I) telle que définie à la revendication 1, répondant à la formule (I'') :

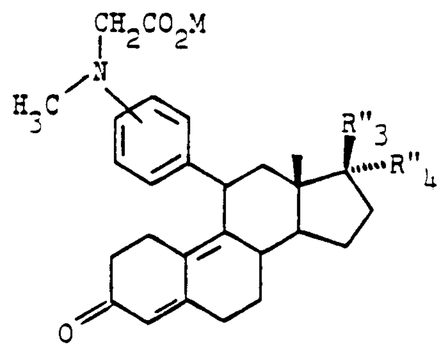

(I")

dans laquelle M est tel que défini précédemment et soit $R''_3$ représente un radical hydroxy et dans ce cas $R''_4$ représente ou bien un groupe -C C-$R_7$ dans lequel les traits pointillés indiquent la présence éventuelle d'une deuxième ou d'une troisième liaison et $R_7$ représente un atome d'hydrogène, un atome d'halogène, un radical triméthylsilyle ou un radical méthyle éventuellement substitué par un ou plusieurs atomes d'halogène, un radical hydroxy ou par un groupe alkyloxy, alkylthio ou alkylamino renfermant de 1 à 4 atomes de carbone, dialkylamino renfermant de 2 à 8 atomes de carbone ou trialkylsilyle renfermant de 3 à 12 atomes de carbone ou bien un radical 2-propynyle ou 2-propényle, soit $R''_3$ et $R''_4$ représentent ensemble avec l'atome auquel ils sont liés un radical

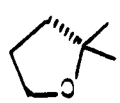

ou

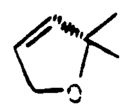

caractérisé en ce que des produits de formule ($II_a$) ou ($II_b$) telle que définie à la revendication 1, répondant à la formule ($II''_a$) et ($II''_b$) :

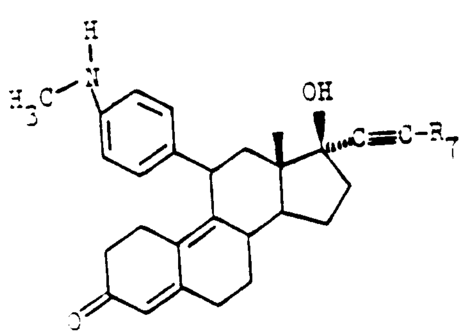

($II''_a$)

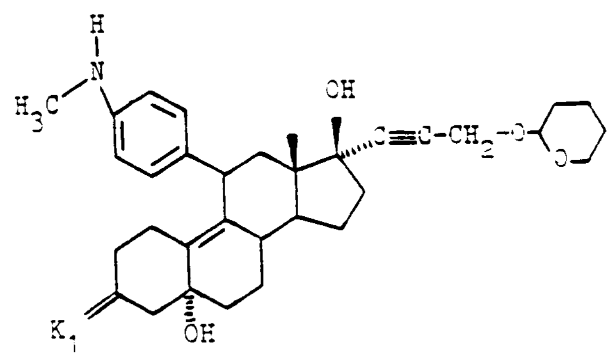

($II''_b$)

dans laquelle $K_1$ représente un groupe oxo protégé sous forme d'un radical éthylènedioxy ou d'un acétal diméthylique sont soumis à l'action du bromoacétate d'éthyle en présence d'une base azotée pour obtenir respectivement les produits de formules ($IV''_a$) et ($IV''_b$) :

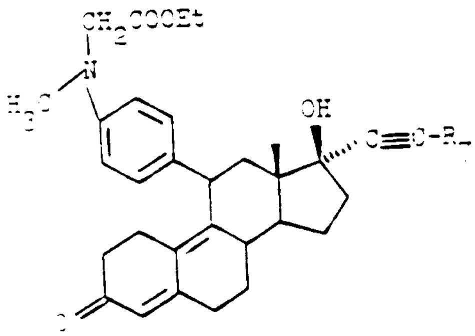

(IV"$_a$)

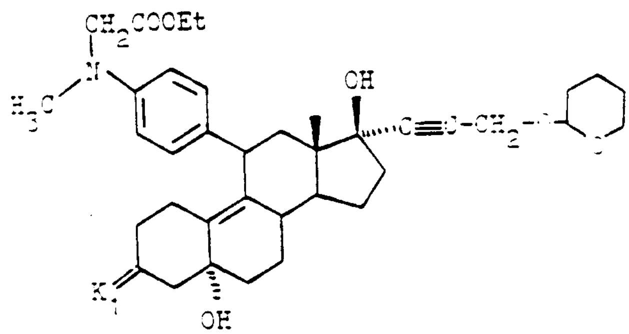

(IV"$_b$)

produits de formules (IV″$_a$) et (IV″$_B$) qui si désiré sont hydrogénés pour obtenir respectivement suivant le catalyseur utilisé soit les produits de formules (V″$_a$) et (V″$_b$) :

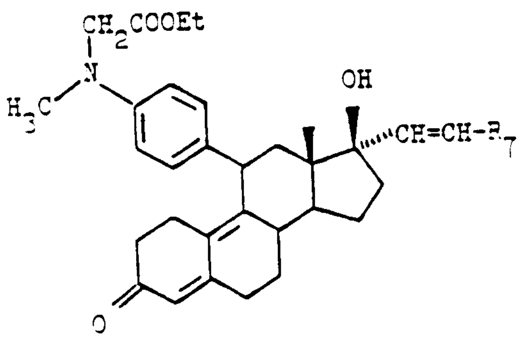

(V"$_a$)

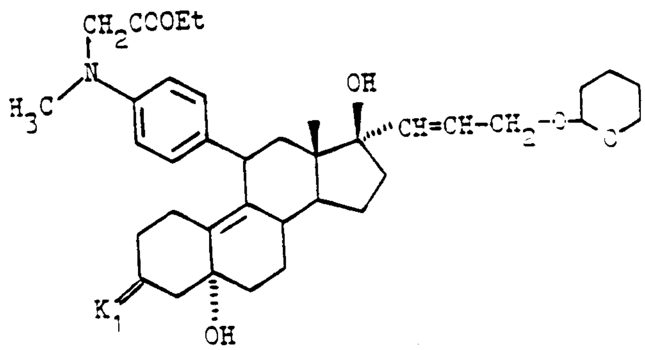

(V"$_b$

soit les produits de formule (VI″$_a$) et (VI″$_b$) :

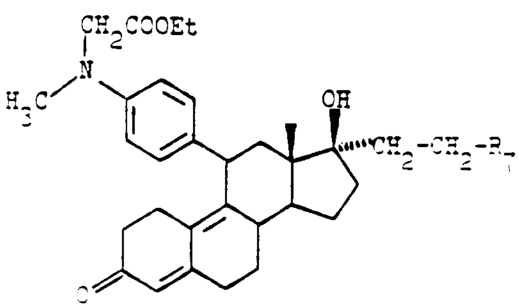

(VI"$_a$)

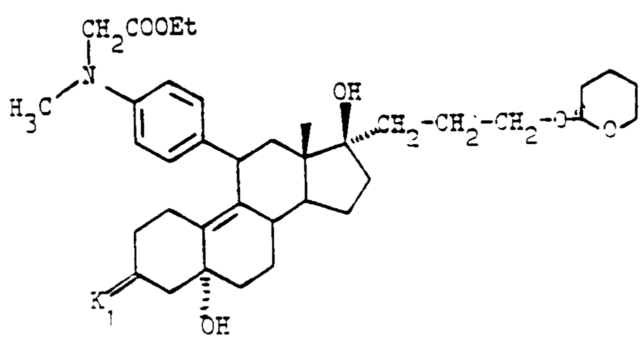

(VI"$_b$)

produits de formule (V''$_b$) et (VI''$_b$) qui par traitement acide conduisent aux produits de formules (V''$_c$) et (VI''$_c$) :

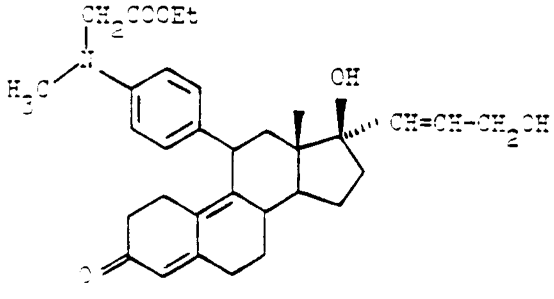

(V"c)

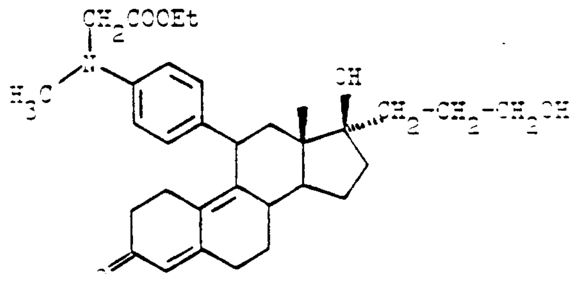

(VI"c)

produits de formules (V"$_c$) et (VI"$_c$) que si désiré l'on fait réagir avec du chlorure de tosyle dans la pyridine pour obtenir respectivement les produits de formules (V"$_d$) et (VI"$_d$) :

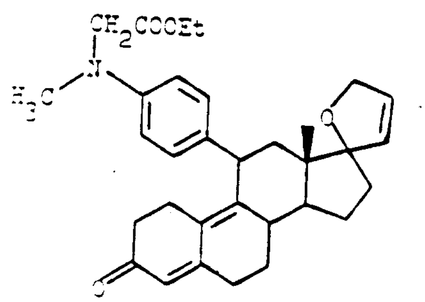

(V"d)

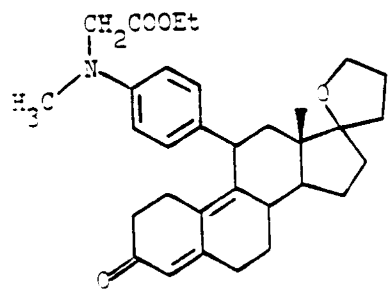

(VI"d)

produits de formules (IV"$_a$), (V"$_a$), (V"$_c$), VI"$_a$), (VI"$_c$, (V"$_d$) et (VI″$_d$) que l'on traite avec une solution hydroalcoolique de soude puis si désiré par l'acide chlorhydrique pour obtenir les produits de formule (I″).

**7.** Procédé selon l'une quelconque des revendication & à 6, caractérisé en ce que l'on prépare le produit de formule (I) telle que définie à la revendication 1, dont le nom suit :
- sel de sodium de l'acide [[4-[17béta hydroxy 3-oxo 17alpha (1-propynyl) estra 4,9-dièn 11-béta-yl] phényl] méthylamino] acétique.

**8.** Procédé de préparation de compositions pharmaceutiques, caractérisé en ce que l'on met à titre de principe actif l'un au moins des produits de formule (I) ou l'un au moins de leurs sels pharmaceutiquement acceptables tels que définis à la revendication 1 sous une forme destinée à cet usage.

**9.** Procédé de préparation de compositions pharmaceutiques, caractérisé en ce que l'on met à titre de principe actif l'un au moins des produits de formule (I′) ou l'un au moins de leurs sels pharmaceutiquement acceptables tels que définis à la revendication 3 sous une forme destinée à cet usage.

**10.** Procédé de préparation de compositions pharmaceutiques, caractérisé en ce que l'on met à titre de principe actif l'un au moins des produits de formule (I″) ou l'un au moins de leurs sels pharmaceutiquement acceptables tels que définis à la revendication 6 sous une forme destinée à cet usage.

**11.** Procédé selon la revendication 8, caractérisé en ce que le principe actif est constitué par le produit nommé à la revendication 7.

**12.** A titre de produits industriels nouveaux, les produits de formules (IV$_a$) et (IV$_b$) telles que définies précédemment.

## Claims

**Claims for the following Contracting States : AT, BE, CH, DE, DK, FR, GB, IT, LI, LU, NL, SE**

**1.** The products of general formula (I):

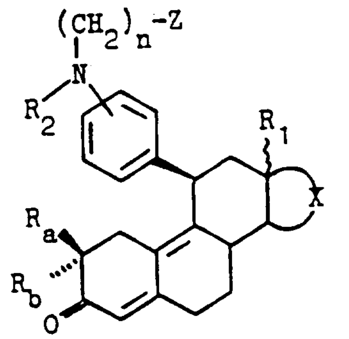

(I)

in which $R_1$ represents an aliphatic hydrocarbon radical containing 1 to 8 carbon atoms, $R_a$ and $R_b$ identical or different represent a hydrogen atom, an alkyl radical containing 1 to 4 carbon atoms, $R_2$ represents a hydrogen atom or an alkyl radical containing 1 to 12 carbon atoms and optionally substituted, n represents one of the values 1 to 6, Z represents a free or salified carboxy group in the form of an alkali metal or an alkaline-earth metal salt, an ammonium salt or an amine salt, X represents the remainder of a pentagonal or hexagonal ring optionally substituted and optionally carrying an unsaturation, and the wavy line in position 13 means that $R_1$ can be found in the alpha or beta position.

**2.** The products of general formula (I) as defined in claim 1 and corresponding to formula (I'):

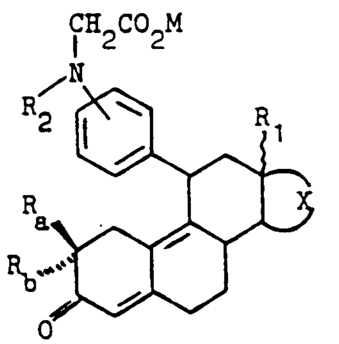

(I')

in which M represents a hydrogen atom or a sodium atom.

**3.** The products of general formula (I) as defined in claims 1 or 2, in which X represents a remainder of formula in which $R_1$ keeps the same meaning as previously, the dotted line in position 16-17 indicates the optional presence of a double bond, Y represents a

$$-\left(\begin{matrix}R_5\\ C\\ R_6\end{matrix}\right)_{n_1}-$$

radical in which $n_1$ represents the number 1 or 2, $R_5$ represents a hydrogen atom, an alkyl radical containing 1 to 8 carbon atoms, an alkenyl or alkynyl radical containing 2 to 8 carbon atoms, an aryl radical containing 6 to 14 carbon atoms or an aralkyl radical containing 7 to 15 carbon atoms, $R_6$ identical to or different from $R_5$ can take one of the values indicated for $R_5$ and can also represent a hydroxyl radical, $R_3$ and $R_4$, identical or different, represent either a hydrogen atom,
or a radical chosen from the group formed by OH, $Oalk_1$, $O\text{-}CO\text{-}alk_2$ radicals in which $alk_1$ and $alk_2$

represent an alkyl radical containing 1 to 8 carbon atoms or an aralkyl radicals containing 7 to 15 carbon atoms,
or an alkyl, alkenyl or alkynyl radical containing 2 to 8 carbon atoms and optionally substituted, or a

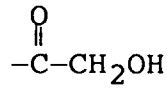

radical,
or a $-COCH_2OCOalk_3$ radical in which $alk_3$ represents an optionally substituted alkyl radical containing 1 to 8 carbon atoms or an aralkyl radical containing 7 to 15 carbon atoms, or a $CO-CO_2H$ radical,
or a $CO-CO-alk_4$ radical in which $alk_4$ represents an alkyl radical containing 1 to 8 carbon atoms,
or a

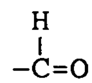

radical,
or a

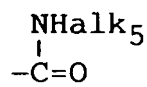

radical, in which $alk_5$ represents an alkyl radical containing 1 to 8 carbon atoms or an aralkyl radical containing 7 to 15 carbon atoms,
or a -C≡N radical,
or $R_3$ and $R_4$ together with the atom to which they are linked form a

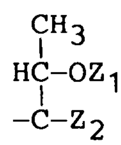

radical in which $Z_1$ represents a hydrogen atom, an alkyl radical or an acyl radical containing 1 to 8 carbon atoms and $Z_2$ an alkyl radical containing 1 to 8 carbon atoms,
or $R_3$ and $R_4$ form together with the atom to which they are linked,
either a

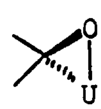

radical
in which U represents a bivalent $-(CH_2)n_2$ radical in which $n_2$ represents an integer equal to 1, 2, 3 or 4 or a bivalent $-CH=CH-(CH_2)n_3-$ radical in which $n_3$ represents an integer equal to 1 or 2,
or a

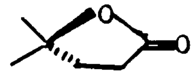

radical

4. The products of general formula (I) as defined in any one of claims 1 to 3, in which the D ring of the steroid nucleus does not carry an unsaturation, $R_5$ and $R_6$ represent a hydrogen atom and $n_1$ is equal to 1.

5. The products of general formula (I) as defined in any one of claims 1 to 4, corresponding to formula (I''):

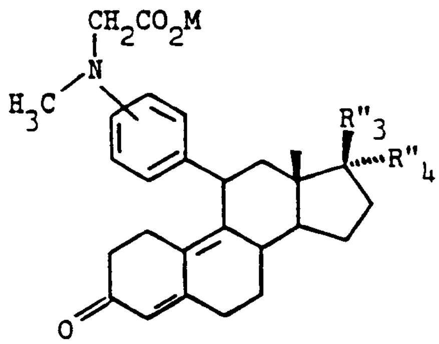

(I'')

in which M is as defined previously and either $R''_3$ represents a hydroxy radical and in this case $R''_4$ represents either a -

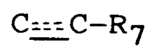

group in which the dotted lines indicate the optional presence of a second or third bond and $R_7$ represents a hydrogen atom, a halogen atom, a trimethylsilyl radical or a methyl radical optionally substituted by one or more halogen atoms, a hydroxy radical or by an alkyloxy, alkylthio or alkylamino group containing 1 to 4 carbon atoms, a dialkylamino group containing 2 to 8 carbon atoms or a trialkylsilyl group containing 3 to 12 carbon atoms or a 2-propynyl or 2-propenyl radical, or $R''_3$ and $R''_4$ represent together with the atom to which they are linked a

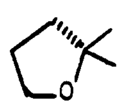 or 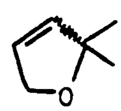

radical

6. The product of general formula (I) as defined in claim 4, whose name follows:
   - sodium salt of [[4-[17beta hydroxy 3-oxo 17alpha (1-propynyl) estra 4,9-dien 11-beta-yl] phenyl] methylamino] acetic acid.

7. Preparation process for the products of formula (I) as defined in claim 1, characterized in that the products of formula ($II_a$) and ($II_b$):

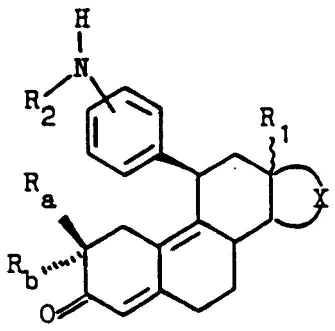

($II_a$)

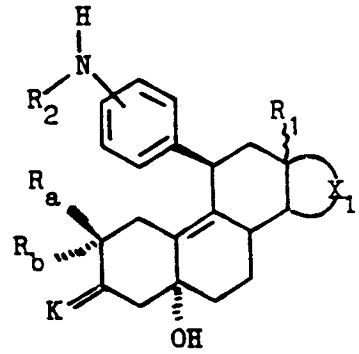

($II_b$)

in which $R_1$, $R_2$, $R_a$, $R_b$ and X are as defined previously, $X_1$ has the meaning indicated in claim 1 for X as well as those values in which the reactive functions are protected and K represents a protected oxo function, are subjected in the presence of a base to the action of a halogenoester of formula (III):

Hal-$(CH_2)$n-$COOR_7$ (III)

in which Hal represents a halogen atom, n has the meaning indicated previously and $R_7$ represents an alkyl radical containing 1 to 4 carbon atoms and optionally substituted by one or more phenyl radicals, in order to obtain the products of formulae ($IV_a$) and ($IV_b$) respectively:

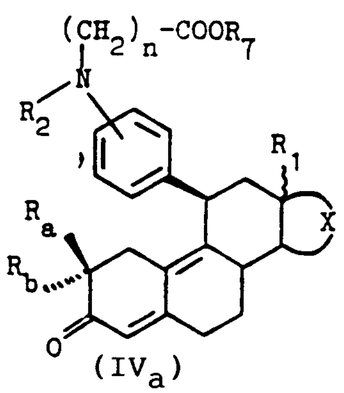

($IV_a$)

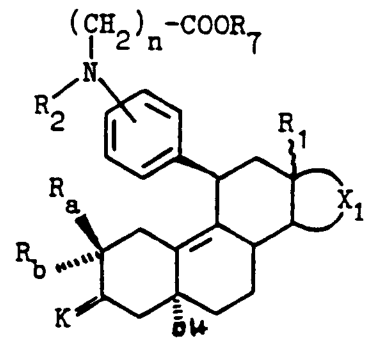

($IV_b$)

which products of formula ($IV_b$) are subjected to an optional dehydration and deprotection reaction of the protected reactive functions in order to obtain the products of formula ($IV_a$) and which products of formula ($IV_a$) are subjected to a basic treatment then, if desired, to an acid treatment in order to obtain the products of formula (I).

**8.** Process according to claim 7, characterized in that the products of formulae ($IV_a$) and ($IV_b$) are subjected, if desired, to one or more of the following reactions in any order:

a) partial or total hydrogenation of the multiple bonds which can be contained by X and $X_a$,

b) cyclization of the two reactive functions carried by X so as to form a spirane ring in position 17 of the steroid nucleus.

**9.** As medicaments, the products of formula (I) as defined in claim 1.

**10.** As medicaments, the products of formula (I) as defined in one of the claims 2 to 6.

**11.** Pharmaceutical compositions containing as active ingredient at least one of the medicaments defined according to claims 9 or 10.

**12.** As new industrial products, the products of formulae ($IV_a$) and ($IV_b$) as defined previously.

**Claims for the following Contracting State : ES**

**1.** Preparation process for the products of general formula (I):

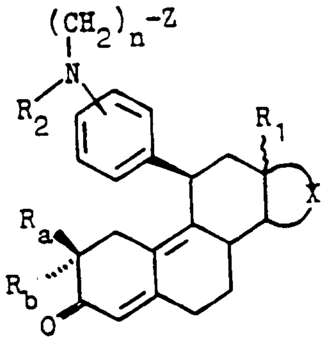

(I)

in which $R_1$ represents an aliphatic hydrocarbon radical containing 1 to 8 carbon atoms, $R_a$ and $R_b$ identical or different represent a hydrogen atom, an alkyl radical containing 1 to 4 carbon atoms, $R_2$ represents a hydrogen atom or an alkyl radical containing 1 to 12 carbon atoms and optionally substituted, n represents one of the values 1 to 6, Z represents a free or salified carboxy group in the form of an alkali metal or an alkaline-earth metal salt, an ammonium salt or an amine salt, X represents the remainder of a pentagonal or hexagonal ring optionally substituted and optionally carrying an unsaturation, and the wavy line in position 13 means that $R_1$ can be found in the alpha or beta position, characterized in that the products of formula ($II_a$) and ($II_b$):

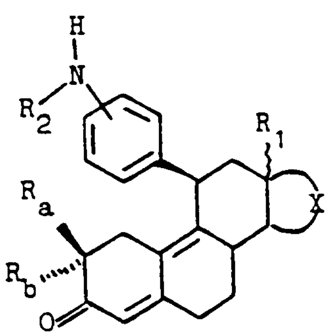

($II_a$)

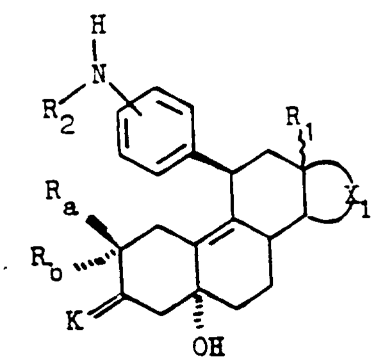

($II_b$)

in which $R_1$, $R_2$, $R_a$, $R_b$ and X are as defined previously, $X_1$ has the meaning indicated in claim 1 for X as well as those values in which the reactive functions are protected and K represents a protected oxo function, are subjected in the presence of a base to the action of a halogenoester of formula (III):

$Hal-(CH_2)n-COOR_7$ (III)

in which Hal represents a halogen atom, n has the meaning indicated previously and $R_7$ represents an alkyl radical containing 1 to 4 carbon atoms and optionally substituted by one or more phenyl radicals, in order to obtain the products of formulae ($IV_a$) and ($IV_b$) respectively:

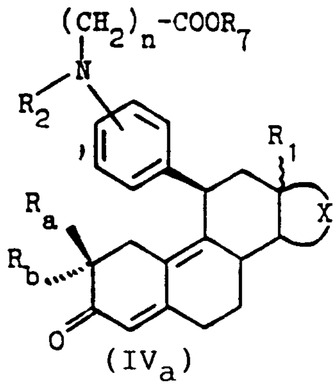

(IVa)

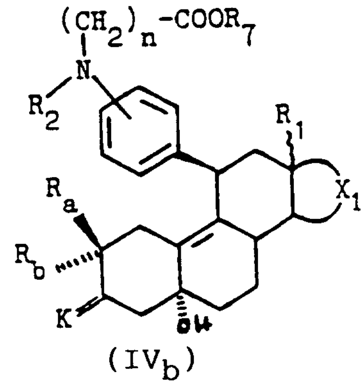

(IVb)

which products of formula ($IV_b$) are subjected to an optional dehydration and deprotection reaction of the protected reactive functions in order to obtain the products of formula ($IV_a$) and which products of formula ($IV_a$) are subjected to a basic treatment then, if desired, to an acid treatment in order to obtain the products of formula (I).

**2.** Process according to claim 1, characterized in that the products of formulae ($IV_a$) and ($IV_b$) are subjected, if desired, to one or more of the following reactions in any order:

a) partial or total hydrogenation of the multiple bonds which can be contained by X and $X_a$,

b) cyclization of the two reactive functions carried by X so as to form a spirane ring in position 17 of the steroid nucleus.

**3.** Process according to claim 1 or 2 for the preparation of the products of general formula (I) as defined in claim 1, corresponding to formula (I'):

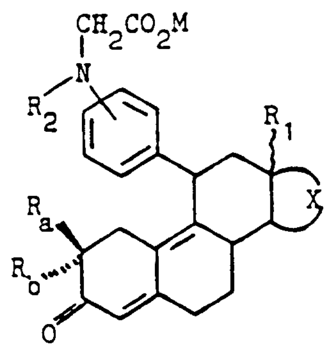

(I')

in which M represents a hydrogen atom or a sodium atom, characterized in that a product of formula (III) in which n = 1 is used at the start.

**4.** Process according to any one of claims 1 to 3, characterized in that a product of formula ($II_a$) or ($II_b$) is used at the start, in which X represents a remainder of formula

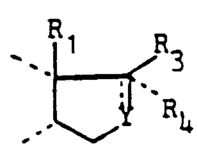

in which $R_1$ keeps the same meaning as previously, the dotted line in position 16-17 indicates the optional presence of a double bond, Y represents a

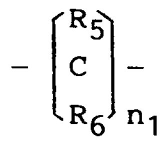

radical in which $n_1$ represents the number 1 or 2, $R_5$ represents a hydrogen atom, an alkyl radical containing 1 to 8 carbon atoms, an alkenyl or alkynyl radical containing 2 to 8 carbon atoms, an aryl radical containing 6 to 14 carbon atoms or an aralkyl radical containing 7 to 15 carbon atoms, $R_6$ identical to or different from $R_5$ can take one of the values indicated for $R_5$ and can also represent a hydroxyl radical, $R_3$ and $R_4$, identical or different, represent either a hydrogen atom,
or a radical chosen from the group formed by OH, $Oalk_1$, $O\text{-}CO\text{-}alk_2$ radicals in which $alk_1$ and $alk_2$ represent an alkyl radical containing 1 to 8 carbon atoms or an aralkyl radical containing 7 to 15 carbon atoms,
or an alkyl, alkenyl or alkynyl radical containing 2 to 8 carbon atoms and optionally substituted,
or a

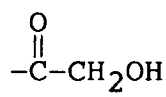

radical,
or a $-COCH_2OCOalk_3$ radical in which $alk_3$ represents an optionally substituted alkyl radical containing 1 to 8 carbon atoms or an aralkyl radical containing 7 to 15 carbon atoms,
or a $CO\text{-}CO_2H$ radical,
or a $CO\text{-}CO\text{-}alk_4$ radical in which $alk_4$ represents an alkyl radical containing 1 to 8 carbon atoms,
or a

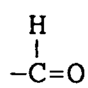

radical,
or a

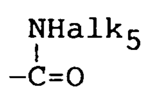

radical, in which $alk_5$ represents an alkyl radical containing 1 to 8 carbon atoms or an aralkyl radical containing 7 to 15 carbon atoms,
or a $-C{\equiv}N$ radical,
or $R_3$ and $R_4$ together with the atom to which they are linked form a

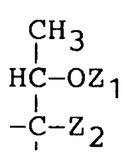

radical
in which $Z_1$ represents a hydrogen atom, an alkyl radical or an acyl radical containing 1 to 8 carbon atoms and $Z_2$ an alkyl radical containing 1 to 8 carbon atoms,
or $R_3$ and $R_4$ form together with the atom to which they are linked,
either a

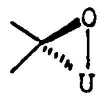

radical

in which U represents a bivalent -$(CH_2)n_2$ radical in which $n_2$ represents an integer equal to 1, 2, 3 or 4 or a bivalent -$CH=CH-(CH_2)n_3$- radical in which $n_3$ represents an integer equal to 1 or 2,

or a

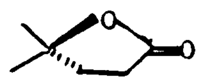

radical

**5.** Process according to claim 4, characterized in that a product of formula ($II_a$) or ($II_b$) is used at the start, in which the D ring of the steroid nucleus does not carry an unsaturation, $R_5$ and $R_6$ represent a hydrogen atom and $n_1$ is equal to 1.

**6.** Process according to any one of claims 1 to 5, for the preparation of the products of general formula (I) as defined in claim 1, corresponding to formula (I''):

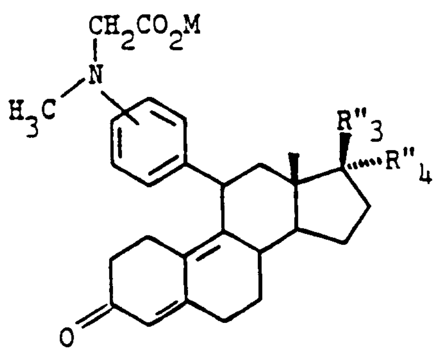

(I'')

in which M is as defined previously and either R''$_3$ represents a hydroxy radical and in this case R''$_4$ represents either a -

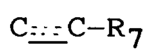

group in which the dotted lines indicate the optional presence of a second or third bond and $R_7$ represents a hydrogen atom, a halogen atom, a trimethylsilyl radical or a methyl radical optionally substituted by one or more halogen atoms, a hydroxy radical or by an alkyloxy, alkylthio or alkylamino group containing 1 to 4 carbon atoms, a dialkylamino group containing 2 to 8 carbon atoms or a trialkylsilyl group containing 3 to 12 carbon atoms or a 2-propynyl or 2-propenyl radical, or R''$_3$ and R''$_4$ represent together with the atom to which they are linked a

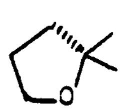 . or 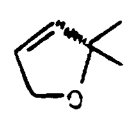

radical

characterized in that the products of formula ($II_a$) or ($II_b$) as defined in claim 1, corresponding to formulae ($II''_a$) and ($II''_b$):

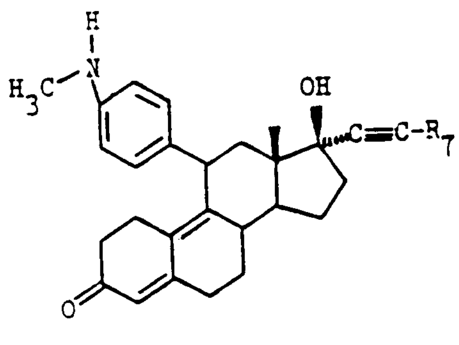

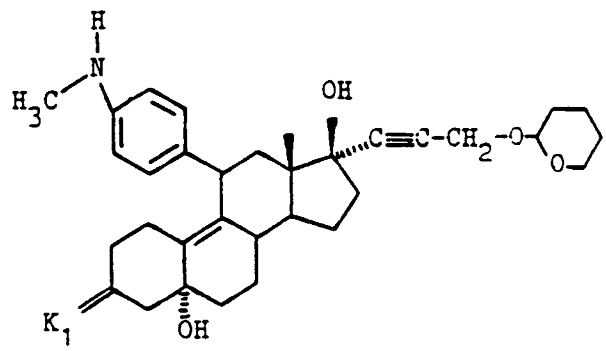

(II"a) (II"b)

in which $K_1$ represents an oxo group protected in the form of an ethylenedioxy or a dimethyl acetal radical are subjected to the action of ethyl bromoacetate in the presence of a nitrogenous base in order to obtain the products of formulae (IV"$_a$) and (IV"$_b$) respectively:

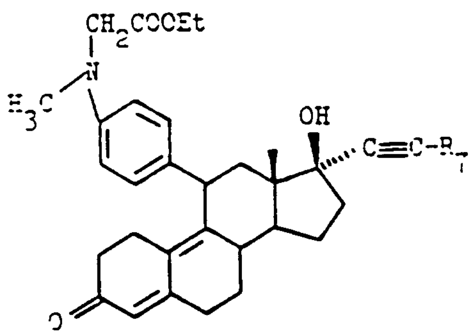

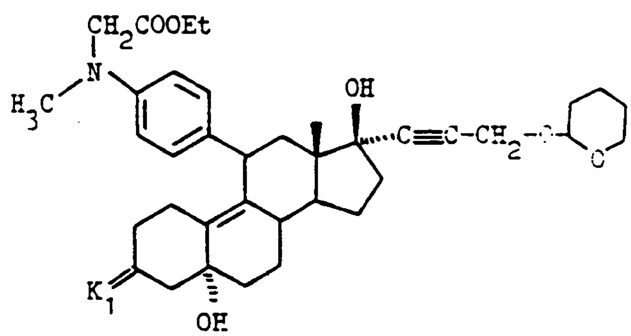

(IV"a) (IV"b)

which products of formulae (IV"$_a$) and (IV"$_b$), if desired, are hydrogenated in order to obtain according to the catalyst used the products of formulae (V"$_a$) and (V"$_b$) respectively:

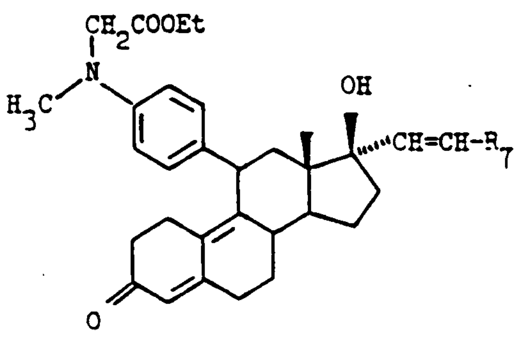

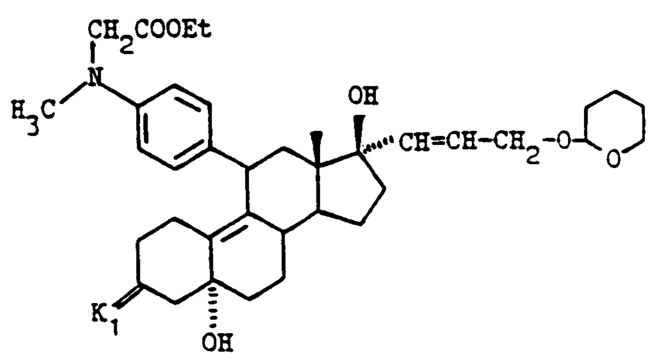

(V"a) (V"b)

or the products of formulae (VI"$_a$) and (VI"$_b$) repectively:

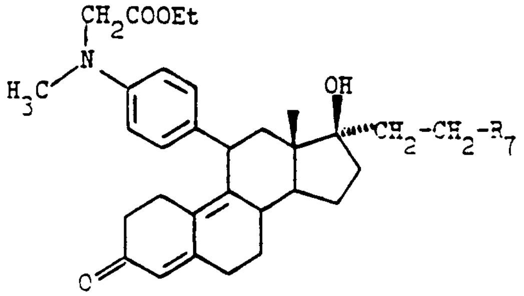

(VI"a)

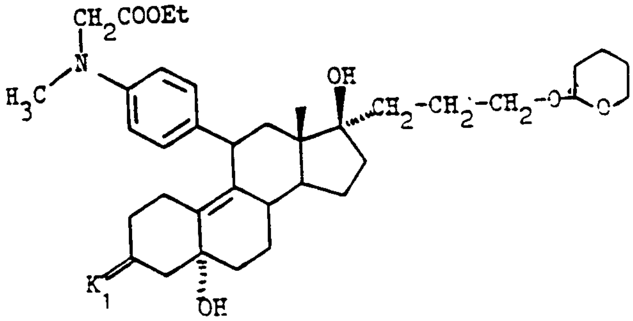

(VI"b)

which products of formulae (V"$_b$) and (VI"$_b$) lead via an acid treatment to the products of formulae (V"$_c$) and (VI"$_c$):

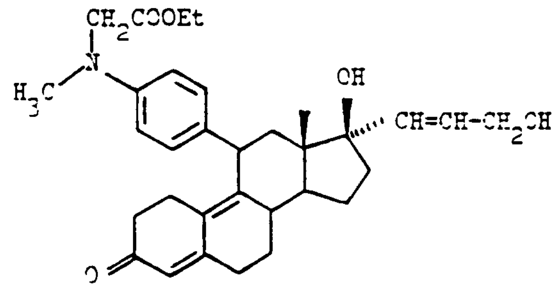

(V"c)

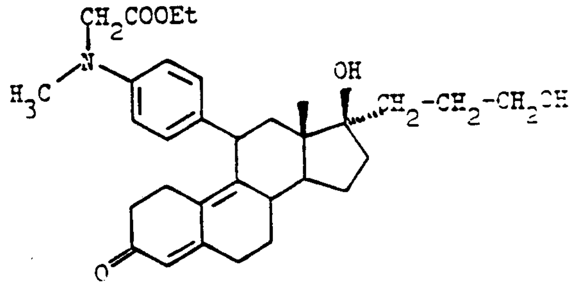

(VI"c):

which products of formulae (V"$_c$) and (VI"$_c$), if desired, are reacted with tosyl chloride in pyridine in order to obtain the products of formulae (V"$_d$) and (VI"$_d$) respectively:

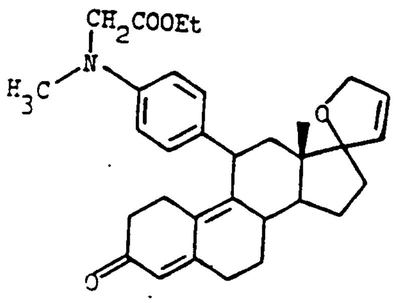

(V"d)

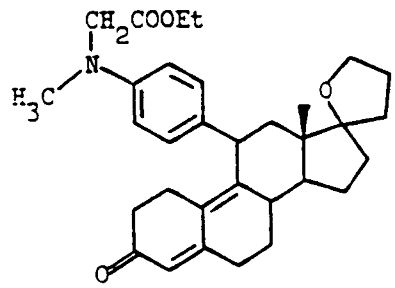

(VI"d)

which products of formulae (IV"$_a$), (V"$_a$), (V"$_c$), (VI"$_a$), (VI"$_c$), (V"$_d$) and (VI"$_d$) are treated with a hydroalcoholic solution of soda, then if desired with hydrochloric acid in order to obtain the products of formula (I").

**Claims for the following Contracting State : GR**

**1.** Preparation process for the products of general formula (I):

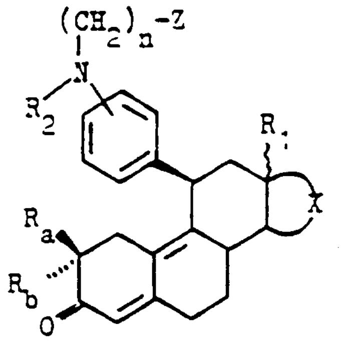

(I)

in which $R_1$ represents an aliphatic hydrocarbon radical containing 1 to 8 carbon atoms, $R_a$ and $R_b$ identical or different represent a hydrogen atom, an alkyl radical containing 1 to 4 carbon atoms, $R_2$ represents a hydrogen atom or an alkyl radical containing 1 to 12 carbon atoms and optionally substituted, n represents one of the values 1 to 6, Z represents a free or salified carboxy group in the form of an alkali metal or an alkaline-earth metal salt, an ammonium salt or an amine salt, X represents the remainder of a pentagonal or hexagonal ring optionally substituted and optionally carrying an unsaturation, and the wavy line in position 13 means that $R_1$ can be found in the alpha or beta position, characterized in that the products of formula ($II_a$) and ($II_b$):

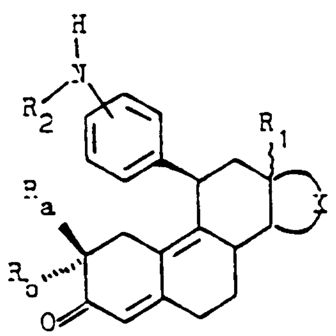

($II_a$)

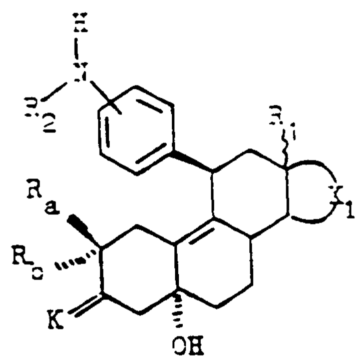

($II_b$)

in which $R_1$, $R_2$, $R_a$, $R_b$ and X are as defined previously, $X_1$ has the meaning indicated in claim 1 for X as well as those values in which the reactive functions are protected and K represents a protected oxo function, are subjected in the presence of a base to the action of a halogenoester of formula (III):

$Hal-(CH_2)n-COOR_7$ (III)

in which Hal represents a halogen atom, n has the meaning indicated previously and $R_7$ represents an alkyl radical containing 1 to 4 carbon atoms and optionally substituted by one or more phenyl radicals, in order to obtain the products of formulae ($IV_a$) and ($IV_b$) respectively:

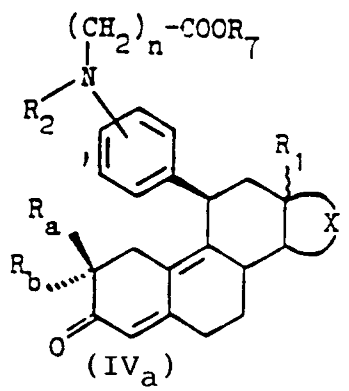

(IVa)

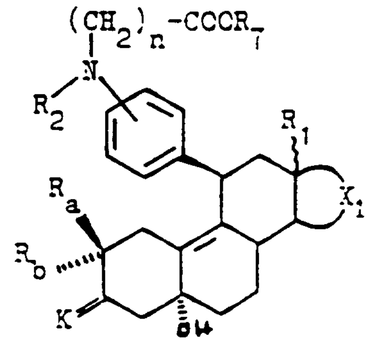

(IVb)

which products of formula ($IV_b$) are subjected to an optional dehydration and deprotection reaction of the protected reactive functions in order to obtain the products of formula ($IV_a$) and which products of formula ($IV_a$) are subjected to a basic treatment then, if desired, to an acid treatment in order to obtain the products of formula (I).

**2.** Process according to claim 1, characterized in that the products of formulae ($IV_a$) and ($IV_b$) are subjected, if desired, to one or more of the following reactions in any order:
  a) partial or total hydrogenation of the multiple bonds which can be contained by X and $X_a$,
  b) cyclization of the two reactive functions carried by X so as to form a spirane ring in position 17 of the steroid nucleus.

**3.** Process according to claim 1 or 2 for the preparation of the products of general formula (I) as defined in claim 1, corresponding to formula (I'):

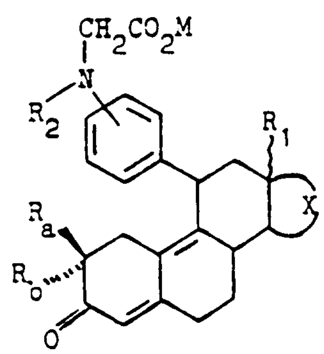

(I')

in which M represents a hydrogen atom or a sodium atom, characterized in that a product of formula (III) in which n = 1 is used at the start.

**4.** Process according to any one of claims 1 to 3, characterized in that a product of formula ($II_a$) or ($II_b$) is used at the start, in which X represents a remainder of formula

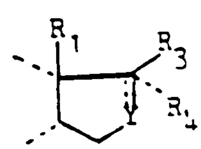

in which $R_1$ keeps the same meaning as previously, the dotted line in position 16-17 indicates the optional presence of a double bond, Y represents a

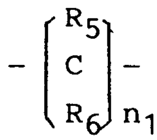

radical in which $n_1$ represents the number 1 or 2,
$R_5$ represents a hydrogen atom, an alkyl radical containing 1 to 8 carbon atoms, an alkenyl or alkynyl radical containing 2 to 8 carbon atoms, an aryl radical containing 6 to 14 carbon atoms or an aralkyl radical containing 7 to 15 carbon atoms, $R_6$ identical to or different from $R_5$ can take one of the values indicated for $R_5$ and can also represent a hydroxyl radical, $R_3$ and $R_4$, identical or different, represent either a hydrogen atom,
or a radical chosen from the group formed by OH, $Oalk_1$, $O\text{-}CO\text{-}alk_2$ radicals in which $alk_1$ and $alk_2$ represent an alkyl radical containing 1 to 8 carbon atoms or an aralkyl radicals containing 7 to 15 carbon atoms,
or an alkyl, alkenyl or alkynyl radical containing 2 to 8 carbon atoms and optionally substituted,
or a

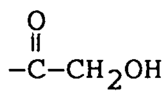

radical,
or a $-COCH_2OCOalk_3$ radical in which $alk_3$ represents an optionally substituted alkyl radical containing 1 to 8 carbon atoms or an aralkyl radical containing 7 to 15 carbon atoms,
or a $CO\text{-}CO_2H$ radical,
or a $CO\text{-}CO\text{-}alk_4$ radical in which $alk_4$ represents an alkyl radical containing 1 to 8 carbon atoms,
or a

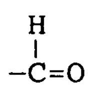

radical,
or a

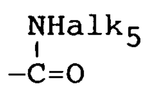

radical, in which $alk_5$ represents an alkyl radical containing 1 to 8 carbon atoms or an aralkyl radical containing 7 to 15 carbon atoms,
or a $-C\equiv N$ radical,
or $R_3$ and $R_4$ together with the atom to which they are linked form a

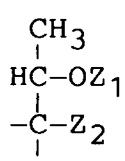

radical
in which $Z_1$ represents a hydrogen atom, an alkyl radical or an acyl radical containing 1 to 8 carbon atoms and $Z_2$ an alkyl radical containing 1 to 8 carbon atoms,
or $R_3$ and $R_4$ form together with the atom to which they are linked,
either a

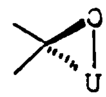

radical

in which U represents a bivalent $-(CH_2)n_2$ radical in which $n_2$ represents an integer equal to 1, 2, 3 or 4 or a bivalent $- CH=CH-(CH_2)n_3-$ radical in which $n_3$ represents an integer equal to 1 or 2,

or a

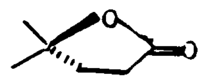

radical

5. Process according to claim 4, characterized in that a product of formula ($II_a$) or ($II_b$) is used at the start, in which the D ring of the steroid nucleus does not carry an unsaturation, $R_5$ and $R_6$ represent a hydrogen atom and $n_1$ is equal to 1.

6. Process according to any one of claims 1 to 5, for the preparation of the products of general formula (I) as defined in claim 1, corresponding to formula (I"):

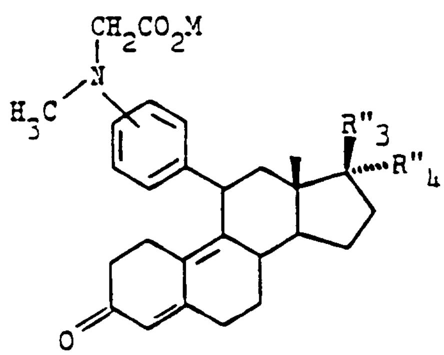

(I")

in which M is as defined previously and either R"$_3$ represents a hydroxy radical and in this case R"$_4$ represents either a -

$$C \equiv C-R_7$$

group in which the dotted lines indicate the optional presence of a second or third bond and $R_7$ represents a hydrogen atom, a halogen atom, a trimethylsilyl radical or a methyl radical optionally substituted by one or more halogen atoms, a hydroxy radical or by an alkyloxy, alkylthio or alkylamino group containing 1 to 4 carbon atoms, a dialkylamino group containing 2 to 8 carbon atoms or a trialkylsilyl radical containing 3 to 12 carbon atoms or a 2-propynyl or 2-propenyl radical, or R"$_3$ and R"$_4$ represent together with the atom to which they are linked a

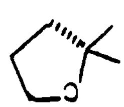

or

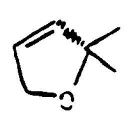

radical

characterized in that the products of formula ($II_a$) or ($II_b$) as defined in claim 1, corresponding to

formulae (II"$_a$) and (II"$_b$):

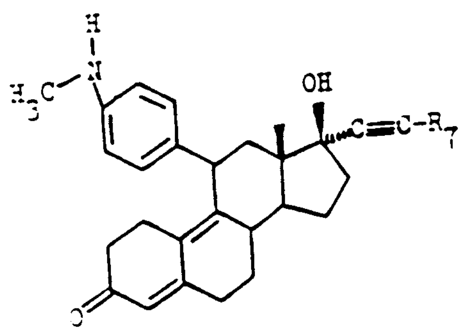

(II"$_a$)

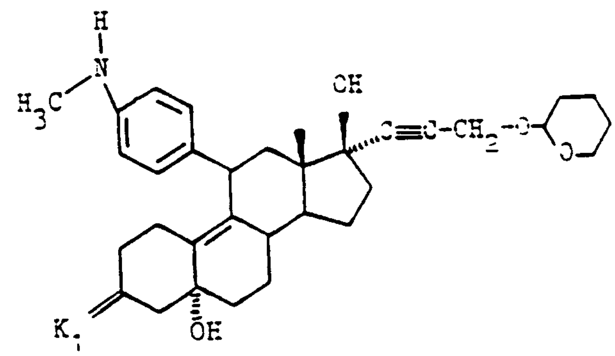

(II"$_b$)

in which $K_1$ represents an oxo group protected in the form of an ethylenedioxy or a dimethyl acetal radical are subjected to the action of ethyl bromoacetate in the presence of a nitrogenous base in order to obtain the products of formulae (IV"$_a$) and (IV"$_b$) respectively:

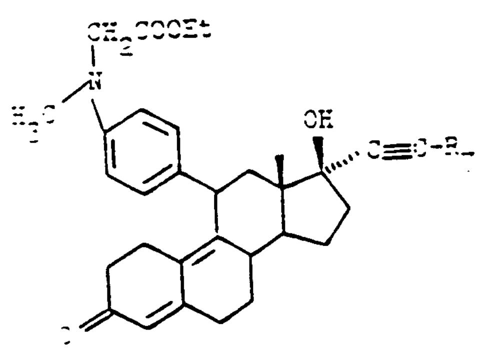

(IV"$_a$)

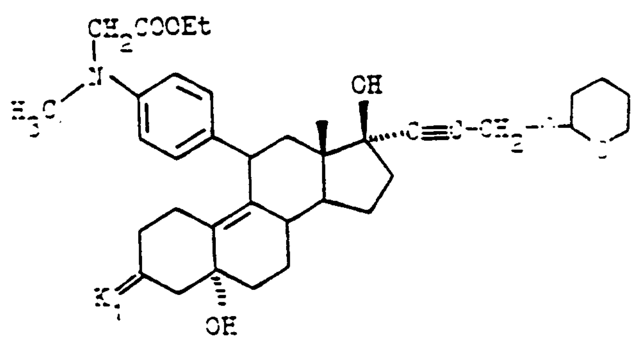

(IV"$_b$)

which products of formulae (IV"$_a$) and (IV"$_b$), if desired, are hydrogenated in order to obtain according to the catalyst used, either the products of formulae (V"$_a$) and (V"$_b$) respectively :

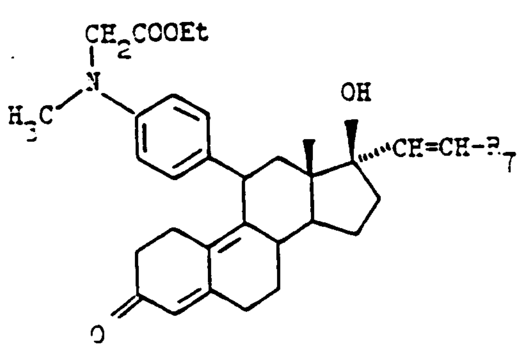

(V"$_a$)

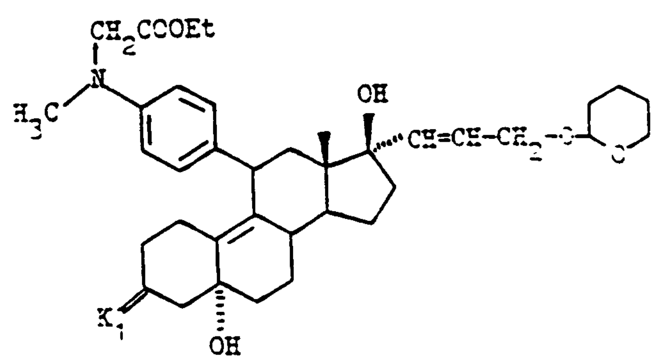

(V"$_b$)

or the products of formulae (VI"$_a$) and (VI"$_b$) respectively:

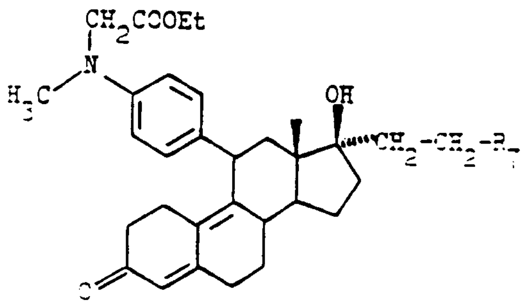

(VI"$_a$)

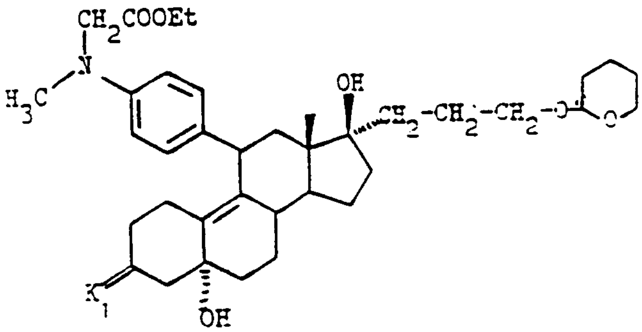

(VI"$_b$)

which products of formulae (V"$_b$) and (VI"$_b$) lead via an acid treatment to the products of formulae (V"$_c$) and (VI"$_c$):

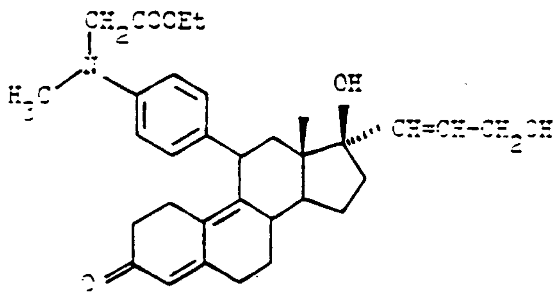

(V"$_c$)

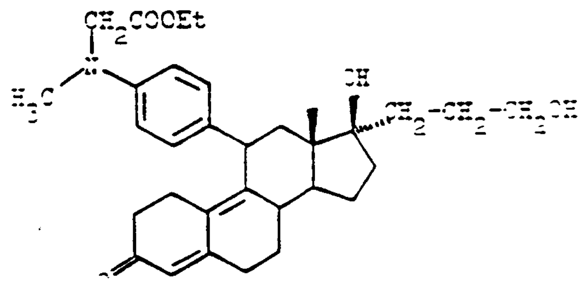

(VI"$_c$):

which products of formulae (V"$_c$) and (VI"$_c$), if desired, are reacted with tosyl chloride in pyridine in order to obtain the products of formulae (V"$_d$) and (VI"$_d$) respectively:

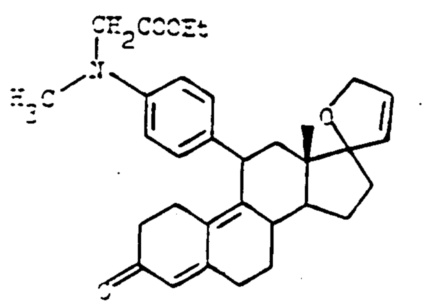

(V"$_d$)

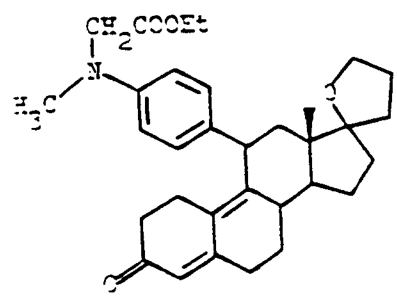

(VI"$_d$)

which products of formulae (IV"$_a$), (V"$_a$), (V"$_c$), (VI"$_a$), (VI"$_c$), (V"$_d$) and (VI"$_d$) are treated with a hydroalcoholic solution of soda, then if desired with hydrochloric acid in order to obtain the products of formula (I").

**7.** Process according to any one of claims 1 to 6, characterized in that the product of formula (I) as defined in claim 1 is prepared, the name of which follows:
- sodium salt of [[4-[17beta hydroxy 3-oxo 17alpha (1-propynyl) estra 4,9-dien 11-beta-yl] phenyl] methylamino] acetic acid.

**8.** Preparation process for pharmaceutical compositions, characterized in that at least one of the products of formula (I) or at least one of their pharmaceutically acceptable salts as defined in claim 1 is used as active ingredient in a form intended for this use.

**9.** Preparation process for pharmaceutical compositions, characterized in that at least one of the products of formula (I') or at least one of their pharmaceutically acceptable salts as defined in claim 3 is used as active ingredient in a form intended for this use.

**10.** Preparation process for pharmaceutical compositions, characterized in that at least one of the products of formula (I'') or at least one of their pharmaceutically acceptable salts as defined in claim 6 is used as active ingredient in a form intended for this use.

**11.** Process according to claim 8, characterized in that the active ingredient is constituted by the product named in claim 7.

**12.** As new industrial products, the products of formulae ($IV_a$) and ($IV_b$) as defined previously.

**Patentansprüche**

**Patentansprüche für folgende Vertragsstaaten : AT, BE, CH, DE, DK, FR, GB, IT, LI, LU, NL, SE**

**1.** Die Produkte der allgemeinen Formel (I)

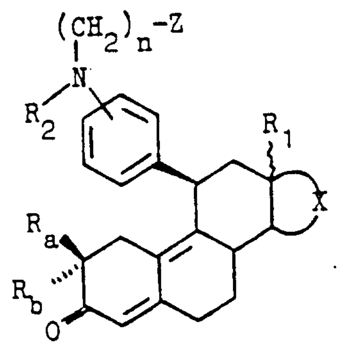

(I)

worin $R_1$ einen aliphatischen Kohlenwasserstoffrest mit 1 bis 8 Kohlenstoffatomen bedeutet, $R_a$ und $R_b$, die identisch oder verschieden sind, bedeuten ein Wasserstoffatom, einen Alkylrest mit 1 bis 4 Kohlenstoffatomen, $R_2$ bedeutet ein Wasserstoffatom oder einen Alkylrest mit 1 bis 12 Kohlenstoffatomen und gegebenenfalls substituiert, n bedeutet einen der Werte 1 bis 6, Z bedeutet eine freie Carboxygruppe oder als Salz in Form eines Alkali- oder Erdalkalimetallsalzes, eines Ammoniumsalzes oder eines Aminsalzes, X bedeutet den Rest eines pentagonalen oder hexagonalen Ringes gegebenenfalls substituiert und gegebenenfalls Träger einer Unsättigung und die Wellenlinie in 13-Stellung bedeutet, daß $R_1$ sich in alpha- oder beta-Stellung befinden kann.

**2.** Die Produkte der allgemeinen Formel (I), wie in Anspruch 1 definiert, und entsprechend der Formel (I'):

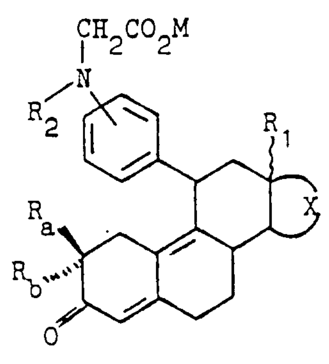

(I')

worin M ein Wasserstoffatom oder ein Natriumatom darstellt.

3. Die Produkte der allgemeinen Formel (I), wie in Anspruch 1 oder 2 definiert, worin X einen Rest der Formel

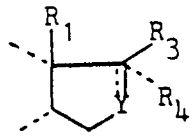

bedeutet, worin $R_1$ dieselbe Bedeutung wie vorstehend beibehält, die punkrierte Linie in 16-17-Stellung symbolisiert

die eventuelle Anwesenheit einer Doppelbindung, Y bedeutet einen Rest

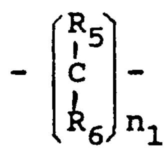

worin $n_1$ die Zahl 1 oder 2 darstellt, $R_5$ ein Wasserstoffatom bedeutet, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen, $R_6$, das mit $R_5$ identisch oder davon verschieden ist, kann eine der für $R_5$ angegebenen Werte annehmen und kann auch einen Hydroxylrest bedeuten, $R_3$ und $R_4$, die identisch oder verschieden sind, bedeuten

entweder ein Wasserstoffatom,

oder einen Rest ausgewählt aus der Gruppe gebildet durch die Reste OH, $Oalc_1$, $O\text{-}CO\text{-}alc_2$, worin $alc_1$ und $alc_2$ einen Alkylrest mit 1 bis 8 Kohlenstoffatomen oder Aralkyl mit 7 bis 15 Kohlenstoffatomen darstellen,

oder einen Alkyl-, Alkenyl- oder Alkinylrest mit 2 bis 8 Kohlenstoffatomen und gegebenenfalls substituiert,

oder einen

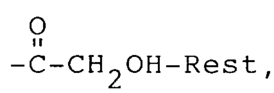

oder einen Rest $-COCH_2OCOalc_3$, worin $alc_3$ einen Alkylrest mit 1 bis 8 Kohlenstoffatomen, gegebenenfalls substituiert, oder einen Aralkylrest mit 7 bis 15 Kohlenstoffatomen bedeutet,

oder einen Rest $CO\text{-}CO_2H$,

oder einen Rest $CO\text{-}CO\text{-}alc_4$, worin $alc_4$ einen Alkylrest mit 1 bis 8 Kohlenstoffatomen bedeutet,

oder einen Rest

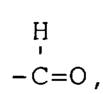

oder einen Rest

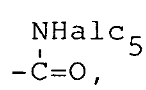

worin $alc_5$ einen Alkylrest mit 1 bis 8 Kohlenstoffatomen oder einen Aralkylrest mit 7 bis 15 Kohlenstoffatomen bedeutet,

oder einen Rest $-C{\equiv}N$,

oder $R_3$ und $R_4$ bilden zusammen mit dem Atom, an das sie gebunden sind, einen Rest

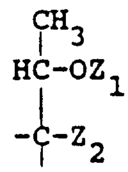

worin $Z_1$ ein Wasserstoffatom, einen Alkylrest oder einen Acylrest mit 1 bis 8 Kohlenstoffatomen bedeutet und $Z_2$ einen Alkylrest mit 1 bis 8 Kohlenstoffatomen darstellt,
oder $R_3$ und $R_4$ bilden zusammen mit dem Atom, an das sie gebunden sind,
entweder einen Rest

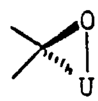

worin U einen zweiwertigen Rest $-(CH_2)n_2$ bedeutet, worin $n_2$ eine ganze Zahl 1, 2, 3 oder 4 ist, oder einen zweiwertigen Rest $-CH=CH-(CH_2)n_3-$, worin $n_3$ eine ganze Zahl gleich 1 oder 2 ist,
oder einen Rest

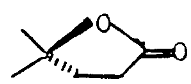

4. Die Produkte der allgemeinen Formel (I), wie in einem der Ansprüche 1 bis 3 definiert, worin der Ring D des Steroidkerns keine Unsättigung trägt, $R_5$ und $R_6$ ein Wasserstoffatom bedeuten und $n_1$ gleich 1 ist.

5. Die Produkte der allgemeinen Formel (I), wie in einem der Ansprüche 1 bis 4 definiert, entsprechend der Formel (I''):

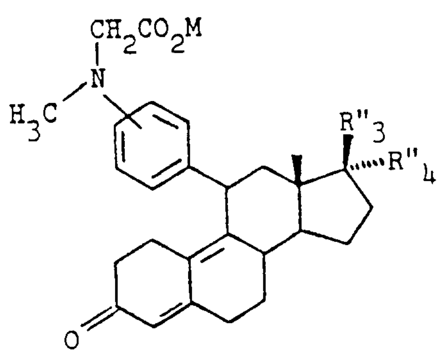

(I'')

worin M wie vorstehend definiert ist, und entweder R''$_3$ bedeutet einen Hydroxyrest und in diesem Fall bedeutet R''$_4$
entweder eine Gruppe

$$-C\equiv C-R_7,$$

worin die gestrichelten Linien die eventuelle Anwesenheit einer zweiten oder dritten Bindung anzeigen und $R_7$ bedeutet ein Wasserstoffatom, ein Halogenatom, einen Trimethylsilylrest oder einen Methylrest gegebenenfalls substituiert durch ein oder mehrere Halogenatome, einen Hydroxyrest oder durch eine Alkyloxy-, Alkylthio- oder Alkylaminogruppe mit 1 bis 4 Kohlenstoffatomen, Dialkylamino mit 2 bis 8 Kohlenstoffatomen oder Trialkylsilyl einschließend 3 bis 12 Kohlenstoffatome
oder einen 2-Propinyl- oder 2-Propenylrest, oder
$R''_3$ und $R''_4$ bedeuten zusammen mit dem Atom an das sie gebunden sind einen Rest

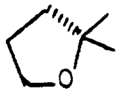

oder

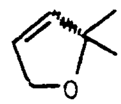

**6.** Das Produkt der allgemeinen Formel (I), wie in Anspruch 4 definiert, dessen Namen folgendermaßen ist:
Natriumsalz der [[4-[17-beta-Hydroxy-3-oxo-17-alpha-(1-propinyl)-estra-4,9-dien-11-beta-yl]-phenyl]-methylamino]-essigsäure.

**7.** Verfahren zur Herstellung der Produkte der Formel (I), wie in Anspruch 1 definiert, dadurch gekennzeichnet, daß die Produkte der Formel ($II_a$) und ($II_b$)

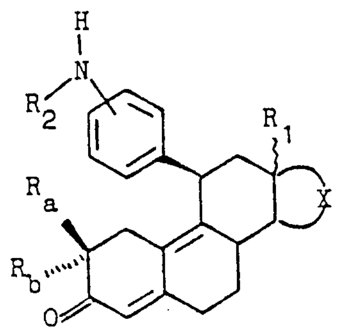

($II_a$)

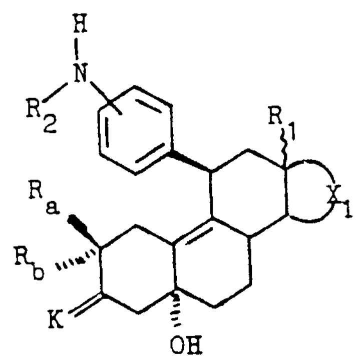

($II_b$)

worin $R_1$, $R_2$, $R_a$, $R_b$ und X wie vorstehend definiert sind, $X_1$ die in Anspruch 1 angegebene Bedeutung für X hat sowie diese Werte, worin die reaktiven Funktionen geschützt sind, und K eine geschützte Oxofunktion darstellt, in Gegenwart einer Base der Einwirkung eines Halogenesters der Formel (III):

$Hal\text{-}(CH_2 n\text{-}COOR_7$ (III)

unterworfen werden, worin Hal ein Halogenatom darstellt, n die vorstehend angegebene Bedeutung hat und $R_7$ einen Alkylrest mit 1 bis 4 Kohlenstoffatomen darstellt und gegebenenfalls durch einen oder mehrere Phenylreste substituiert ist, um die Produkte der Formeln ($IV_a$) bzw. ($IV_b$)

$(IV_b)$ :

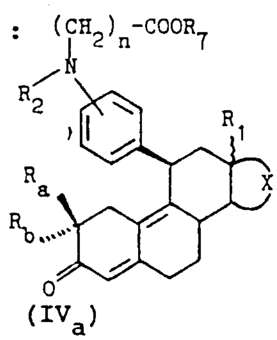

$(IV_a)$

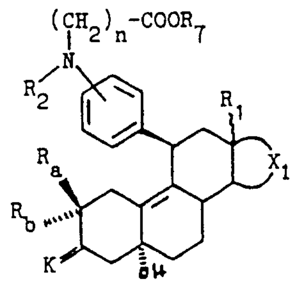

$(IV_b)$

zu erhalten, wobei die Produkte der Formel ($IV_b$), die man einer Dehydratationsreaktion und gegebenenfalls Schutzgruppenabspaltung der geschützten reaktiven Funktionen unterwirft, um die Produkte der Formel ($IV_a$) zu erhalten und die Produkte der Formel ($IV_a$), welche man einer basischen Behandlung und dann gewünschtenfalls einer sauren Behandlung unterwirft, um die Produkte der Formel (I) zu erhalten.

**8.** Verfahren gemäß Anspruch 7, dadurch gekennzeichnet, daß die Produkte der Formel ($IV_a$) und ($IV_b$) gewünschtenfalls einer oder mehreren der folgenden Reaktionen in irgendeiner Reihenfolge unterworfen werden:

a) partielle oder vollständige Hydrierung der Mehrfachbindungen, welche X und $X_a$ tragen können;

b) Cyclisierung der zwei reaktiven Funktionen, welche von X getragen sind derart, daß ein Spiranring in 17-Stellung des Steroidkerns gebildet wird.

**9.** Als Arzneimittel die Produkte der Formel (I), wie in Anspruch 1 definiert.

**10.** Als Arzneimittel die Produkte der Formel (I), wie in einem der Ansprüche 2 bis 6 definiert.

**11.** Pharmazeutische Zusammensetzungen, enthaltend als Wirkstoff wenigstens eines der Arzneimittel, die gemäß den Ansprüchen 9 oder 10 definiert sind.

**12.** Als neue industrielle Produkte die Produkte der Formeln ($IV_a$) und ($IV_b$), wie vorstehend definiert.

**Patentansprüche für folgenden Vertragsstaat : ES**

**1.** Verfahren zur Herstellung der Produkte der allgemeinen Formel (I)

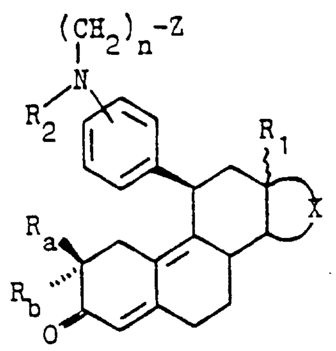

(I)

worin $R_1$ einen aliphatischen Kohlenwasserstoffrest mit 1 bis 8 Kohlenstoffatomen bedeutet, $R_a$ und $R_b$, die identisch oder verschieden sind, bedeuten ein Wasserstoffatom, einen Alkylrest mit 1 bis 4 Kohlenstoffatomen, $R_2$ bedeutet ein Wasserstoffatom oder einen Alkylrest mit 1 bis 12 Kohlenstoffatomen und gegebenenfalls substituiert, n bedeutet einen der Werte 1 bis 6, Z bedeutet eine freie

Carboxygruppe oder als Salz in Form eines Alkali- oder Erdalkalimetallsalzes, eines Ammoniumsalzes oder eines Aminsalzes, X bedeutet den Rest eines pentagonalen oder hexagonalen Ringes gegebenenfalls substituiert und gegebenenfalls Träger einer Unsättigung und die Wellenlinie in 13-Stellung bedeutet, daß $R_1$ sich in alpha- oder beta-Stellung befinden kann, dadurch gekennzeichnet, daß die Produkte der Formel ($II_a$) und ($II_b$):

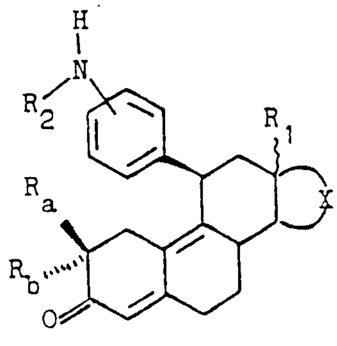

($II_a$)

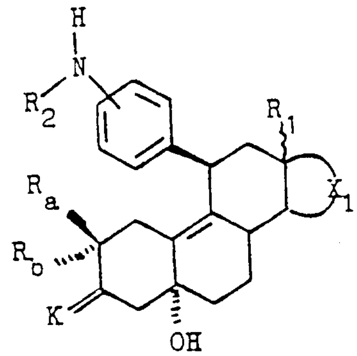

($II_b$)

worin $R_1$, $R_2$, $R_a$, $R_b$ und X wie vorstehend definiert sind, $X_1$ die in Anspruch 1 für X angegebene Bedeutung hat sowie diese Bedeutungen, worin die reaktiven Funktionen geschützt sind, und K eine geschützte Oxofunktion darstellt, in Gegenwart einer Base der Einwirkung eines Halogenesters der Formel (III):

$Hal-(CH_2)n-COOR_7$ (III)

unterworfen werden, worin Hal ein Halogenatom bedeutet, n die vorstehend angegebene Bedeutung hat, und $R_7$ einen Alkylrest mit 1 bis 4 Kohlenstoffatomen darstellt und gegebenenfalls durch einen oder mehrere Phenylreste substituiert ist, um die Produkte der Formeln ($IV_a$) bzw. ($IV_b$)

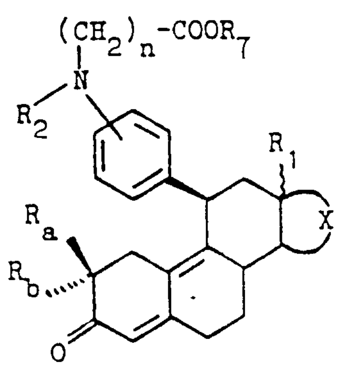

($IV_a$)

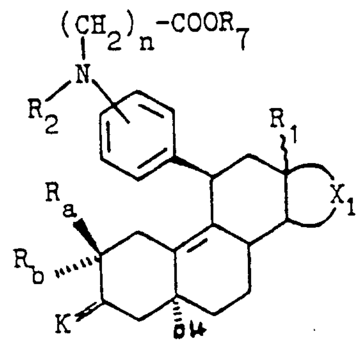

($IV_b$)

zu erhalten, Produkte der Formel ($IV_b$), die man einer Dehydratisierungsreaktion und gegebenenfalls Schutzgruppenabspaltung der geschützten reaktiven Funktionen unterwirft, um die Produkte der Formel ($IV_a$) zu erhalten, und Produkte der Formel ($IV_a$), die man einer basischen und gewünschtenfalls einer sauren Behandlung unterwirft, um die Produkte der Formel (I) zu erhalten.

**2.** Verfahren gemäß Anspruch 1, dadurch gekennzeichnet, daß die Produkte der Formeln ($IV_a$) und ($IV_b$) gewünschtenfalls einer oder mehreren der folgenden Reaktionen in irgendeiner Reihenfolge unterworfen werden:

a) partielle oder vollständige Hydrierung der Mehrfachbindungen, welche X und $X_a$ tragen können;
b) Cyclisierung der zwei reaktiven Funktionen, welche von X getragen sind derart, daß ein Spiranring in 17-Stellung des Steroidkerns gebildet wird.

3. Verfahren gemäß Anspruch 1 oder 2 zur Herstellung der Produkte der Formel (I), wie in Anspruch 1 definiert, entsprechend der Formel (I'):

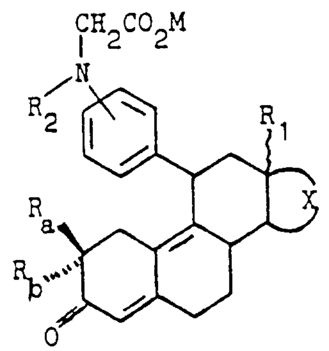

(I')

worin M ein Wasserstoffatom oder ein Natriumatom darstellt, dadurch gekennzeichnet, daß man zu Beginn ein Produkt der Formel (III) verwendet, worin n = 1 ist.

4. Verfahren gemäß einem der Ansprüche 1 bis 3, dadurch gekennzeichnet, daß man zu Beginn ein Produkt der Formel ($II_a$) oder ($II_b$) verwendet, worin X einen Rest der Formel

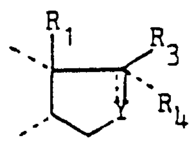

bedeutet, worin $R_1$ dieselbe Bedeutung wie vorstehend beibehält, die punktierte Linie in 16-17-Stellung die eventuelle Anwesenheit einer Doppelbindung symbolisiert, Y bedeutet einen Rest

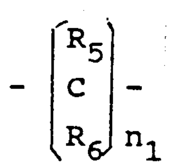

worin $n_1$ die Zahl 1 oder 2 darstellt, $R_5$ ein Wasserstoffatom bedeutet, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen, $R_6$, das mit $R_5$ identisch oder davon verschieden ist, kann eine der für $R_5$ angegebenen Werte annehmen und kann auch einen Hydroxylrest bedeuten, $R_3$ und $R_4$, die identisch oder verschieden sind, bedeuten
entweder ein Wasserstoffatom,
oder einen Rest ausgewählt aus der Gruppe gebildet durch die Reste OH, $Oalc_1$, O-CO-$alc_2$, worin $alc_1$ und $alc_2$ einen Alkylrest mit 1 bis 8 Kohlenstoffatomen oder Aralkyl mit 7 bis 15 Kohlenstoffatomen darstellen,
oder einen Alkyl-, Alkenyl- oder Alkinylrest mit 2 bis 8 Kohlenstoffatomen und gegebenenfalls substituiert,
oder einen

$$-\overset{O}{\overset{\|}{C}}-CH_2OH\text{-Rest,}$$

oder einen Rest $-COCH_2OCOalc_3$, worin $alc_3$ einen Alkylrest mit 1 bis 8 Kohlenstoffatomen, gegebenenfalls substituiert, oder einen Aralkylrest mit 7 bis 15 Kohlenstoffatomen bedeutet,
oder einen Rest $CO-CO_2H$,
oder einen Rest $CO-CO-alc_4$, worin $alc_4$ einen Alkylrest mit 1 bis 8 Kohlenstoffatomen bedeutet,
oder einen Rest

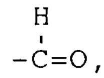

oder einen Rest

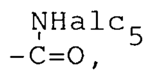

worin $alc_5$ einen Alkylrest mit 1 bis 8 Kohlenstoffatomen oder einen Aralkylrest mit 7 bis 15 Kohlenstoffatomen bedeutet,
oder einen Rest -C≡N,
oder $R_3$ und $R_4$ bilden zusammen mit dem Atom,
an das sie gebunden sind, einen Rest

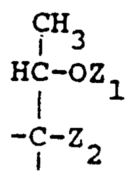

worin $Z_1$ ein Wasserstoffatom, einen Alkylrest oder einen Acylrest mit 1 bis 8 Kohlenstoffatomen bedeutet und $Z_2$ einen Alkylrest mit 1 bis 8 Kohlenstoffatomen darstellt,
oder $R_3$ und $R_4$ bilden zusammen mit dem Atom, an das sie gebunden sind,
entweder einen Rest

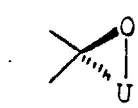

worin U einen zweiwertigen Rest $-(CH_2)n_2$ bedeutet, worin $n_2$ eine ganze Zahl 1, 2, 3 oder 4 ist, oder einen zweiwertigen Rest $-CH=CH-(CH_2)n_3-$, worin $n_3$ eine ganze Zahl gleich 1 oder 2 ist,
oder einen Rest

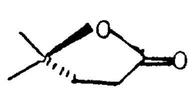

**5.** Verfahren gemäß Anspruch 4, dadurch gekennzeichnet, daß man zu Beginn ein Produkt der Formel ($II_a$) oder ($II_b$) verwendet, worin der Ring D des Steroidkerns keine Unsättigung trägt, $R_5$ und $R_6$ ein Wasserstoffatom bedeuten und $n_1$ gleich 1 ist.

6. Verfahren gemäß einem der Ansprüche 1 bis 5 zur Herstellung der Produkte der Formel (I), wie in Anspruch 1 definiert, entsprechend der Formel (I"):

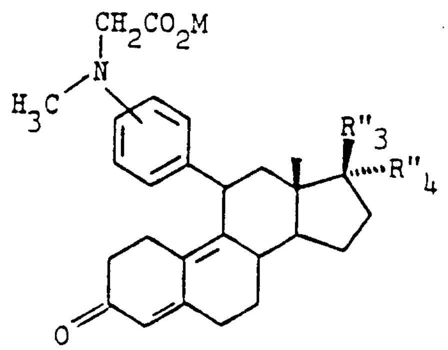

(I")

worin M wie vorstehend definiert ist, und entweder R"$_3$ bedeutet einen Hydroxyrest und in diesem Fall bedeutet R"$_4$

entweder eine Gruppe

$$-C \equiv C-R_7,$$

worin die gestrichelten Linien die eventuelle Anwesenheit einer zweiten oder dritten Bindung anzeigen und $R_7$ bedeutet ein Wasserstoffatom, ein Halogenatom, einen Trimethylsilylrest oder einen Methylrest gegebenenfalls substituiert durch ein oder mehrere Halogenatome, einen Hydroxyrest oder durch eine Alkyloxy-, Alkylthio- oder Alkylaminogruppe mit 1 bis 4 Kohlenstoffatomen, Dialkylamino mit 2 bis 8 Kohlenstoffatomen oder Trialkylsilyl einschließend 3 bis 12 Kohlenstoffatome

oder einen 2-Propinyl- oder 2-Propenylrest, oder

R"$_3$ und R"$_4$ bedeuten zusammen mit dem Atom, an das sie gebunden sind, einen Rest

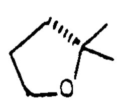

oder

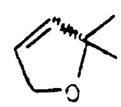

dadurch gekennzeichnet, daß Produkte der Formel ($II_a$) oder ($II_b$) wie in Anspruch 1 definiert, entsprechend der Formel (II"$_a$) oder (II"$_b$):

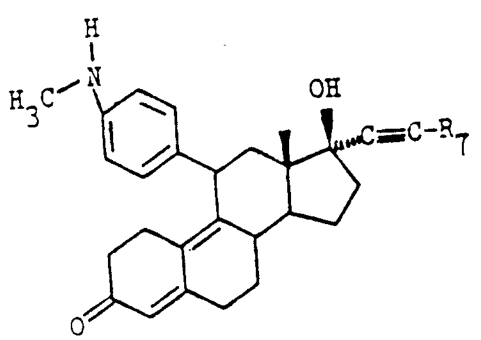

(II"$_a$)

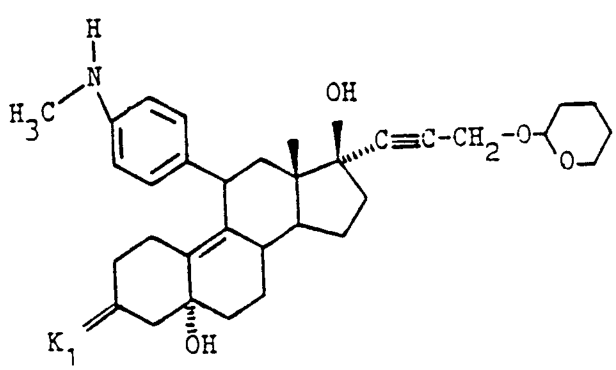

(II"$_b$)

worin $K_1$ eine geschützte Oxogruppe in Form eines Ethylendioxyrestes oder eines Dimethylacetals bedeutet, der Einwirkung von Bromessigsäureethylester in Anwesenheit einer Stickstoffbase unterworfen werden, um die Produkte der Formel (IV"$_a$) bzw. (IV"$_b$):

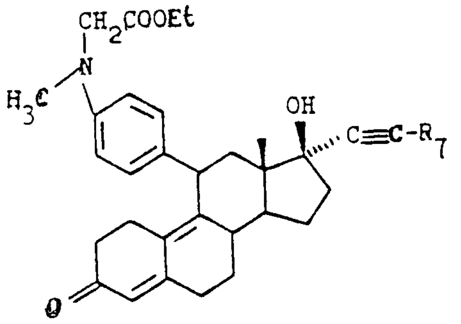

(IV"$_a$)

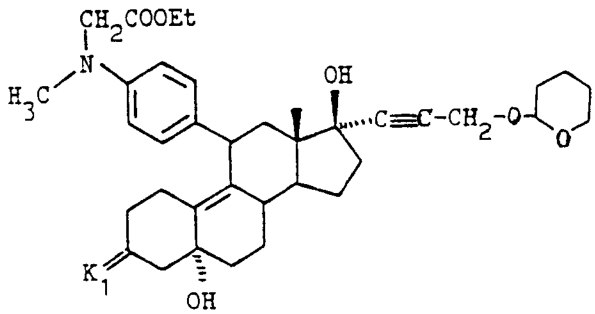

(IV"$_b$)

zu erhalten, Produkte der Formeln (IV"$_a$) und (IV"$_b$), welche gewünschtenfalls hydriert werden, um je nach dem verwendeten Katalysator entweder die Produkte der Formeln (V"$_a$) bzw. (V"$_b$):

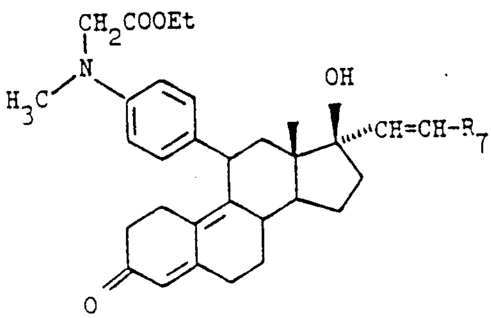

(V"$_a$)

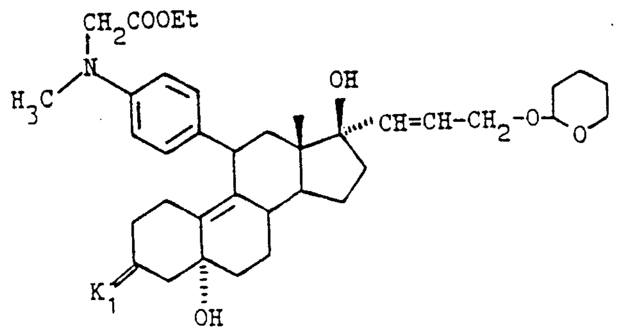

(V"$_b$

zu erhalten, oder die Produkte der Formeln (VI"$_a$) bzw. (VI"$_b$):

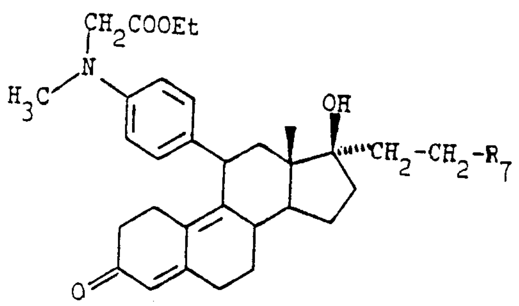

(VI"$_a$)

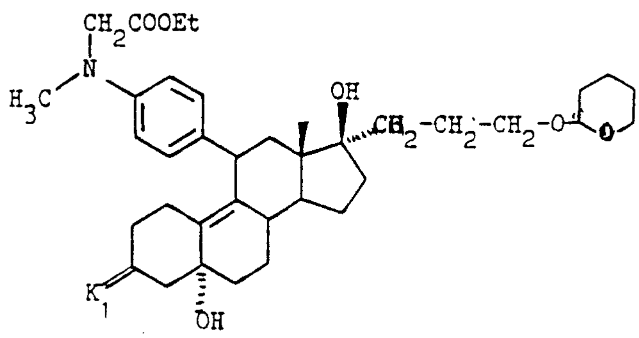

(VI"$_b$)

Produkte der Formel (V"$_b$) und (VI"$_b$), die durch saure Behandlung zu Produkten der Formeln (V"$_c$) und (VI"$_c$):

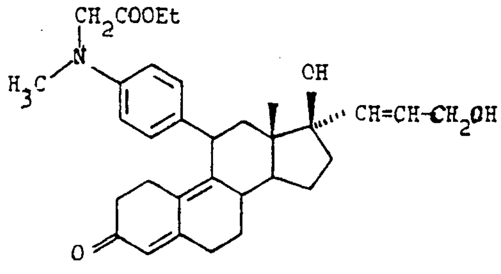

(V"c)

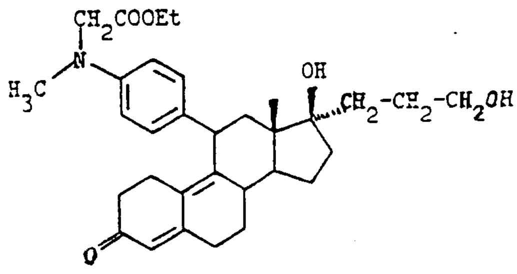

(VI"c)

führen, Produkte der Formeln ($V''_c$) und ($VI''_c$), die man gewünschtenfalls mit Tosylchlorid in Pyridin reagieren läßt, um die Produkte der Formeln ($V''_d$) bzw. ($VI''_d$):

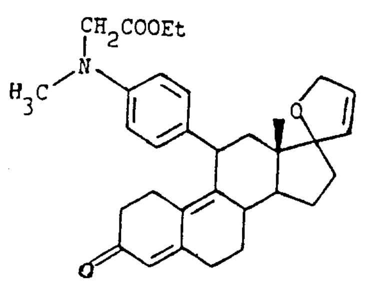

(V"d)

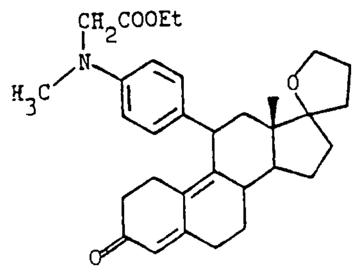

(VI"d)

zu erhalten, Produkte der Formeln ($IV''_a$), ($V''_a$), ($V''_c$), ($VI''_a$), ($VI''_c$), ($V''_d$) und ($VI''_d$), die man mit einer wäßrig-alkoholischen Natriumhydroxidlösung und dann gewünschtenfalls mit Salzsäure behandelt, um die Produkte der Formel (I") zu erhalten.

**Patentansprüche für folgenden Vertragsstaat : GR**

**1.** Verfahren zur Herstellung der Produkte der allgemeinen Formel (I)

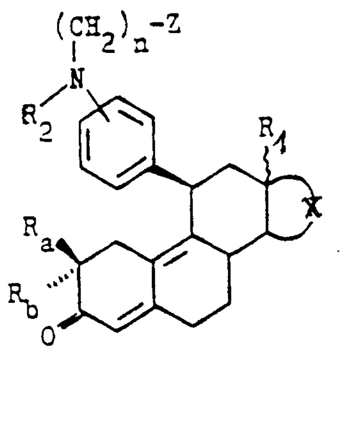

(I)

worin $R_1$ einen aliphatischen Kohlenwasserstoffrest mit 1 bis 8 Kohlenstoffatomen bedeutet, $R_a$ und $R_b$, die identisch oder verschieden sind, bedeuten ein Wasserstoffatom, einen Alkylrest mit 1 bis 4 Kohlenstoffatomen, $R_2$ bedeutet ein Wasserstoffatom oder einen Alkylrest mit 1 bis 12 Kohlenstoffatomen und gegebenenfalls substituiert, n bedeutet einen der Werte 1 bis 6, Z bedeutet eine freie Carboxygruppe oder als Salz in Form eines Alkali- oder Erdalkalimetallsalzes, eines Ammoniumsalzes

oder eines Aminsalzes, X bedeutet den Rest eines pentagonalen oder hexagonalen Ringes gegebenenfalls substituiert und gegebenenfalls Träger einer Unsättigung und die Wellenlinie in 13-Stellung bedeutet, daß $R_1$ sich in alpha- oder beta-Stellung befinden kann, dadurch gekennzeichnet, daß die Produkte der Formel ($II_a$) und ($II_b$):

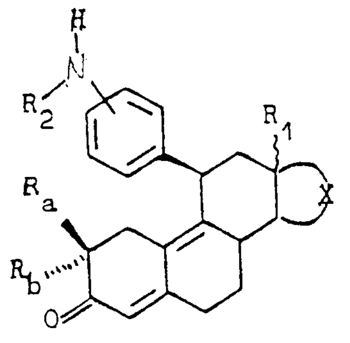

($II_a$)

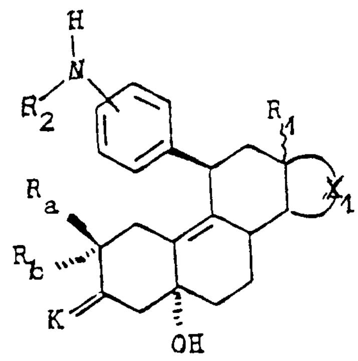

($II_b$)

worin $R_1$, $R_2$, $R_a$, $R_b$ und X wie vorstehend definiert sind, $X_1$ die in Anspruch 1 für X angegebene Bedeutung hat sowie diese Bedeutungen, worin die reaktiven Funktionen geschützt sind, und K eine geschützte Oxofunktion darstellt, in Gegenwart einer Base der Einwirkung eines Halogenesters der Formel (III):

$Hal-(CH_2)n-COOR_7$ (III)

unterworfen werden, worin Hal ein Halogenatom bedeutet, n die vorstehend angegebene Bedeutung hat, und $R_7$ einen Alkylrest mit 1 bis 4 Kohlenstoffatomen darstellt und gegebenenfalls durch einen oder mehrere Phenylreste substituiert ist, um die Produkte der Formeln ($IV_a$) bzw. ($IV_b$)

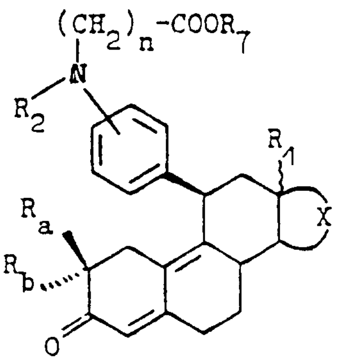

($IV_a$)

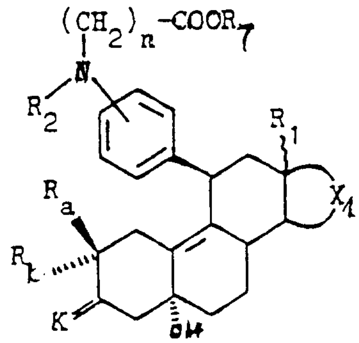

($IV_b$)

zu erhalten, Produkte der Formel ($IV_b$), die man einer Dehydratisierungsreaktion und gegebenenfalls Schutzgruppenabspaltung der geschützten reaktiven Funktionen unterwirft, um die Produkte der Formel ($IV_a$) zu erhalten, und Produkte der Formel ($IV_a$), die man einer basischen und gewünschtenfalls einer sauren Behandlung unterwirft, um die Produkte der Formel (I) zu erhalten.

**2.** Verfahren gemäß Anspruch 1, dadurch gekennzeichnet, daß die Produkte der Formeln ($IV_a$) und ($IV_b$) gewünschtenfalls einer oder mehreren der folgenden Reaktionen in irgendeiner Reihenfolge unterworfen werden:

a) partielle oder vollständige Hydrierung der Mehrfachbindungen, welche X und $X_a$ tragen können;
b) Cyclisierung der zwei reaktiven Funktionen, welche von X getragen sind derart, daß ein Spiranring in 17-Stellung des Steroidkerns gebildet wird.

**3.** Verfahren gemäß Anspruch 1 oder 2 zur Herstellung der Produkte der Formel (I), wie in Anspruch 1 definiert, entsprechend der Formel (I'):

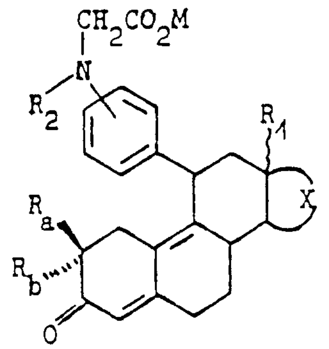

(I')

worin M ein Wasserstoffatom oder ein Natriumatom darstellt, dadurch gekennzeichnet, daß man zu Beginn ein Produkt der Formel (III) verwendet, worin n = 1 ist.

**4.** Verfahren gemäß einem der Ansprüche 1 bis 3, dadurch gekennzeichnet, daß man zu Beginn ein Produkt der Formel ($II_a$) oder ($II_b$) verwendet, w

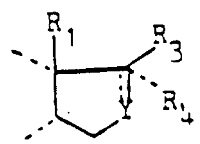

orin X einen Rest der Formel bedeutet, worin $R_1$ dieselbe Bedeutung wie vorstehend beibehält, die punktierte Linie in 16-17-Stellung die eventuelle Anwesenheit einer Doppelbindung symbolisiert, Y bedeutet einen Rest

$$-\left(\begin{matrix} R_5 \\ C \\ R_6 \end{matrix}\right)_{n_1}-$$

worin $n_1$ die Zahl 1 oder 2 darstellt, $R_5$ ein Wasserstoffatom bedeutet, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen, $R_6$, das mit $R_5$ identisch oder davon verschieden ist, kann eine der für $R_5$ angegebenen Werte annehmen und kann auch einen Hydroxylrest bedeuten, $R_3$ und $R_4$, die identisch oder verschieden sind, bedeuten
entweder ein Wasserstoffatom,
oder einen Rest ausgewählt aus der Gruppe gebildet durch die Reste OH, $Oalc_1$, O-CO-$alc_2$, worin $alc_1$ und $alc_2$ einen Alkylrest mit 1 bis 8 Kohlenstoffatomen oder Aralkyl mit 7 bis 15 Kohlenstoffatomen darstellen,
oder einen Alkyl-, Alkenyl- oder Alkinylrest mit 2 bis 8 Kohlenstoffatomen und gegebenenfalls substituiert,
oder einen

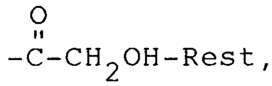

oder einen Rest $-COCH_2OCOalc_3$, worin $alc_3$ einen Alkylrest mit 1 bis 8 Kohlenstoffatomen, gegebenenfalls substituiert, oder einen Aralkylrest mit 7 bis 15 Kohlenstoffatomen bedeutet,
oder einen Rest $CO\text{-}CO_2H$,
oder einen Rest CO-CO-$alc_4$, worin $alc_4$ einen Alkylrest mit 1 bis 8 Kohlenstoffatomen bedeutet,
oder einen Rest

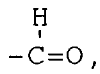

oder einen Rest

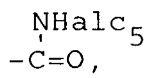

worin $alc_5$ einen Alkylrest mit 1 bis 8 Kohlenstoffatomen oder einen Aralkylrest mit 7 bis 15 Kohlenstoffatomen bedeutet,
oder einen Rest -C≡N,
oder $R_3$ und $R_4$ bilden zusammen mit dem Atom, an das sie gebunden sind, einen Rest

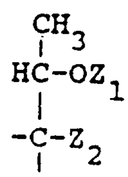

worin $Z_1$ ein Wasserstoffatom, einen Alkylrest oder einen Acylrest mit 1 bis 8 Kohlenstoffatomen bedeutet und $Z_2$ einen Alkylrest mit 1 bis 8 Kohlenstoffatomen darstellt, oder $R_3$ und $R_4$ bilden zusammen mit dem Atom, an das sie gebunden sind,
entweder einen Rest

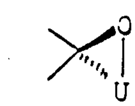

worin U einen zweiwertigen Rest $-(CH_2)n_2$ bedeutet, worin $n_2$ eine ganze Zahl von 1, 2, 3 oder 4 ist, oder einen zweiwertigen Rest $-CH=CH-(CH_2)n_3$, worin $n_3$ eine ganze Zahl gleich 1 oder 2 ist,
oder einen Rest

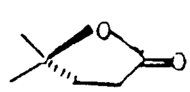

**5.** Verfahren gemäß Anspruch 4, dadurch gekennzeichnet, daß man zu Beginn ein Produkt der Formel ($II_a$) oder ($II_b$) verwendet, worin der Ring D des Steroidkerns keine Unsättigung trägt, $R_5$ und $R_6$ ein Wasserstoffatom bedeuten und $n_1$ gleich 1 ist.

**6.** Verfahren gemäß einem der Ansprüche 1 bis 5 zur Herstellung der Produkte der Formel (I), wie in Anspruch 1 definiert, entsprechend der Formel (I"):

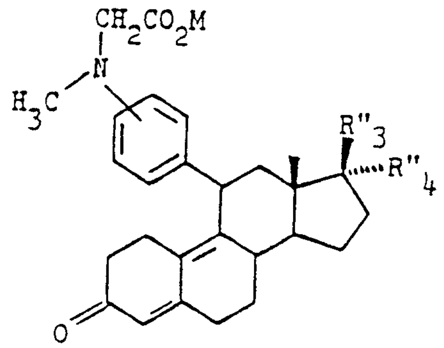

(I")

worin M wie vorstehend definiert ist, und entweder $R''_3$ bedeutet einen Hydroxyrest und in diesem Fall bedeutet $R''_4$
entweder eine Gruppe

$$-C \equiv C-R_7,$$

worin die gestrichelten Linien die eventuelle Anwesenheit einer zweiten oder dritten Bindung anzeigen und $R_7$ bedeutet ein Wasserstoffatom, ein Halogenatom, einen Trimethylsilylrest oder einen Methylrest gegebenenfalls substituiert durch ein oder mehrere Halogenatome, einen Hydroxyrest oder durch eine Alkyloxy-, Alkylthio- oder Alkylaminogruppe mit 1 bis 4 Kohlenstoffatomen, Dialkylamino mit 2 bis 8 Kohlenstoffatomen oder Trialkylsilyl einschließend 3 bis 12 Kohlenstoffatome
oder einen 2-Propinyl- oder 2-Propenylrest, oder
$R''_3$ und $R''_4$ bedeuten zusammen mit dem Atom, an das sie gebunden sind, einen Rest

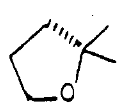

oder

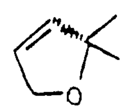

dadurch gekennzeichnet, daß Produkte der Formel ($II_a$) oder ($II_b$), wie in Anspruch 1 definiert, entsprechend der Formel ($II''_a$) oder ($II''_b$):

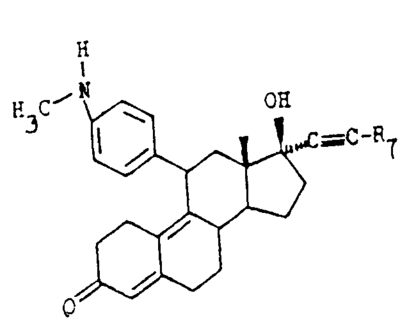

($II''_a$)

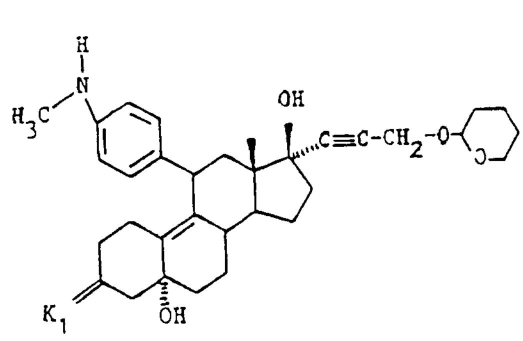

($II''_b$)

worin $K_1$ eine geschützte Oxogruppe in Form eines Ethylendioxyrestes oder eines Dimethylacetals bedeutet, der Einwirkung von Bromessigsäureethylester in Anwesenheit einer Stickstoffbase unterworfen werden, um die Produkte der Formel (IV"$_a$) bzw. (IV"$_b$):

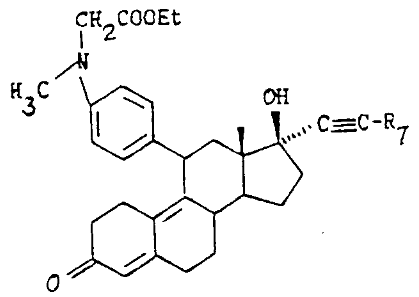

(IV"$_a$)

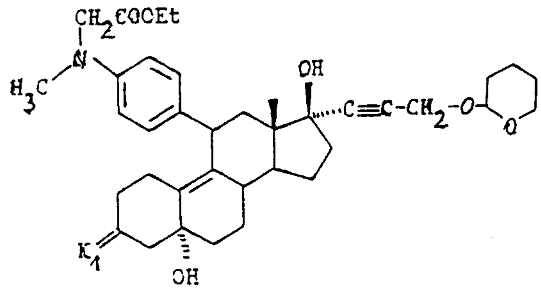

(IV"$_b$)

zu erhalten, Produkte der Formeln (IV"$_a$) und (IV"$_b$), welche gewünschtenfalls hydriert werden, um je nach dem verwendeten Katalysator entweder die Produkte der Formeln (V"$_a$) bzw. (V"$_b$):

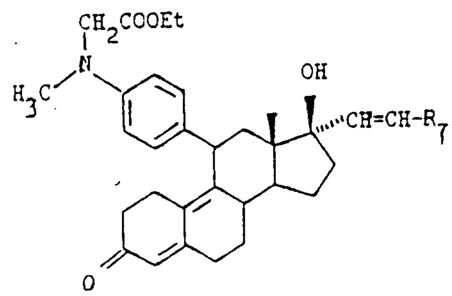

(V"$_a$)

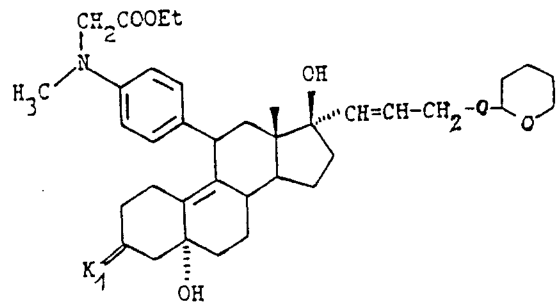

(V"$_b$

zu erhalten, oder die Produkte der Formeln (VI"$_a$) bzw. (VI"$_b$):

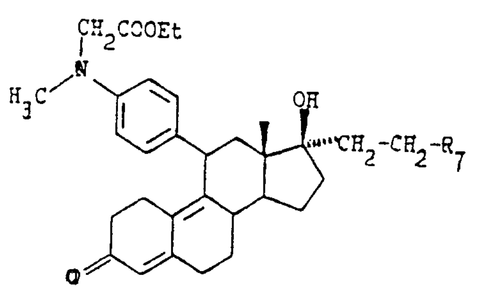

(VI"$_a$)

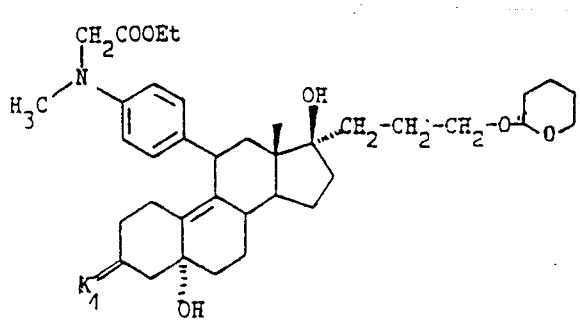

(VI"$_b$)

Produkte der Formel (V"$_b$) und (VI"$_b$), die durch saure Behandlung zu Produkten der Formeln (V"$_c$) und (VI"$_c$):

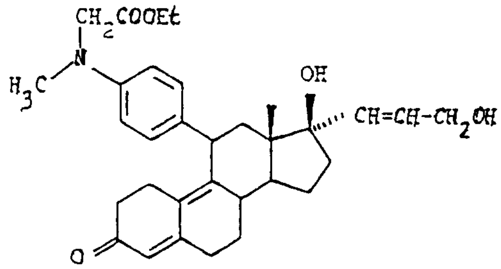

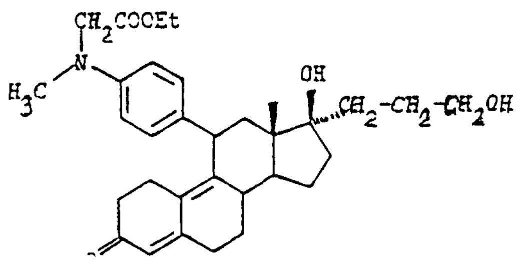

$(V''_c)$ $(VI''_c)$

führen, Produkte der Formeln ($V''_c$) und ($VI''_c$), die man gewünschtenfalls mit Tosylchlorid in Pyridin reagieren läßt, um die Produkte der Formeln ($V''_d$) bzw. ($VI''_d$):

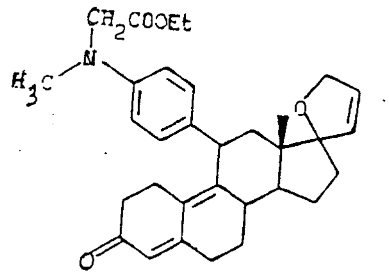

$(V''_d)$

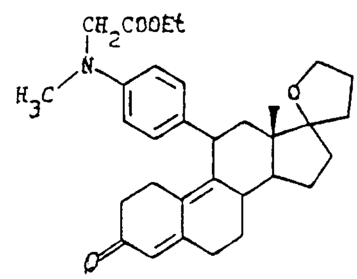

$(VI''_d)$

zu erhalten, Produkte der Formeln ($IV''_a$), ($V''_a$), ($V''_c$), ($VI''_a$), ($VI''_c$), ($V''_d$) und ($VI''_d$), die man mit einer wäßrig-alkoholischen Natriumhydroxidlösung und dann gewünschtenfalls mit Salzsäure behandelt, um die Produkte der Formel (I'') zu erhalten.

**7.** Verfahren gemäß einem der Ansprüche 1 bis 6, dadurch gekennzeichnet, daß man das Produkt der Formel (I) herstellt wie in Anspruch 1 definiert, dessen Namen folgendermaßen ist:
- Natriumsalz der [[4-[17-beta-Hydroxy-3-oxo-17-alpha-(1-propinyl)-estra-4,9-dien-11-beta-yl]-phenyl]-methylamino]-essigsäure.

**8.** Verfahren zur Herstellung von pharmazeutischen Zusammensetzungen, dadurch gekennzeichnet, daß man als Wirkstoff mindestens eines der Produkte der Formel (I) oder mindestens eines seiner pharmazeutisch annehmbaren Salze wie in Anspruch 1 definiert, in einer Form, die für diesen Verwendungszweck bestimmt ist, einsetzt.

**9.** Verfahren zur Herstellung von pharmazeutischen Zusammensetzungen, dadurch gekennzeichnet, daß man als Wirkstoff mindestens eines der Produkte der Formel (I') oder mindestens eines seiner pharmazeutisch annehmbaren Salze, wie in Anspruch 3 definiert, in einer Form, die für diesen Verwendungszweck bestimmt ist, einsetzt.

**10.** Verfahren zur Herstellung von pharmazeutischen Zusammensetzungen, dadurch gekennzeichnet, daß man als Wirkstoff mindestens eines der Produkte der Formel (I'') oder mindestens eines seiner pharmazeutisch annehmbaren Salze, wie in Anspruch 6 definiert, in einer Form, die für diesen Verwendungszweck bestimmt ist, einsetzt.

**11.** Verfahren gemäß Anspruch 8, dadurch gekennzeichnet, daß der Wirkstoff aus dem in Anspruch 7 definierten Produkt besteht.

**12.** Als neue industrielle Produkte, die Produkte der Formeln ($IV_a$) und ($IV_b$) wie vorstehend definiert.